# EUROPEAN PATENT APPLICATION

(11) **EP 4 596 686 A1**
(43) Date of publication of application: **06.08.2025**
(21) Application number: 23872643.4
(22) Date of filing: 29.09.2023
(51) Int. Cl.: C12N 5/10, C12N 15/09, C12N 15/113, C12N 15/12, C12N 15/55, C12N 15/63, C12N 15/86

(54) **SEARCHING METHOD FOR FUNCTIONAL MOLECULE FOR CAUSING RESPONSE IN CELL**

(30) Priority: 29.09.2022 JP 2022156368
(71) Applicant: Keio University, Tokyo, 108-8345 (JP); JSR Corporation, Tokyo 105-8640 (JP)
(72) Inventor: SAYA, Hideyuki, Tokyo 160-8582 (JP); KATO, Shoichi, Tokyo 105-8640 (JP); IKEMOTO, Atsushi, Tokyo 105-8640 (JP)
(74) Representative: TBK
(86) International application number: PCT/JP2023/035799
(87) International publication number: WO 2024/071424

(57) **Abstract**

A vector or set of vectors for analyzing a function of a functional molecule includes a polynucleotide encoding an expression system of a candidate molecule of a functional molecule, a polynucleotide encoding a transcriptional regulatory sequence or translational regulatory sequence that is activated by a predetermined response in a cell, and a polynucleotide encoding a reporter system operably linked to the transcriptional regulatory sequence or translational regulatory sequence. A method for searching a functional molecule causing a response in a cell includes introducing the vector or set of vectors into a cell, and measuring expression of a reporter system included in the vector or set of vectors in the cell.

## Description

### Technical Field

The present invention relates to a method for searching a functional molecule causing a response in a cell, a vector or a set of vectors used in the searching method, and various applications of the searching method.

### Background Art

In various physiological processes such as disease, development, and differentiation, it is important to clarify the function of each gene in search of an effective drug discovery target and understanding of a molecular mechanism of the physiological process.

The functional analysis of a gene includes a method of analyzing an mRNA amount or a protein amount of a target gene in a cell in a specific state or performing post-translational modification of a protein. As a more direct functional analysis method, phenotypic analysis by artificial expression inhibition or increased expression of a target gene is used. As a general phenotypic analysis method, there is a method of selectively inhibiting or activating an arbitrary target gene, and analyzing the effect at the cellular level, the tissue level, or the individual level. However, in the conventional phenotypic analysis, basically, only one kind of gene or any combination of one set of genes can be examined under one experimental condition, and therefore comprehensive analysis of phenotypes requires enormous labor and time.

In recent years, in order to realize comprehensive phenotypic analysis, a screening method combining an RNAi or CRISPR/Cas9 system and a massively parallel sequencer technology has been developed (Non Patent Literature 1 and Non Patent Literature 2). In this technique, shRNA or gRNA vectors having an inhibitory effect on separate genes are pooled and introduced into cells and tissues, thereby causing separate gene inhibition in each cell under the same experimental conditions. Thereafter, cells exhibiting or not exhibiting a phenotype are concentrated, and the abundance of shRNA or gRNA in the obtained cell population is quantified by a massively parallel sequencer, whereby the responsible gene involved in a specific phenotype can be screened. Alternatively, there is a method for realizing comprehensive phenotypic analysis by analyzing a single cell sequence of a cell without concentrating the cell by a phenotype, and simultaneously analyzing a gRNA sequence and gene expression in the single cell (Non Patent Literature 3 to Non Patent Literature 5). Phenotypic analysis combining such gene inhibition and massively parallel sequencer technology has achieved functional analysis of genes with higher throughput compared to conventional phenotypic analysis. However, these analysis methods also require long-term culture in order to concentrate the cell population, or require time and effort in concentration operation and library preparation of a single cell sequence, and thus there is room for further improvement.

As a tool for functional analysis of genes, a lentiviral vector that co-expresses DNA encoding shRNA and a reporter gene that visualizes introduction of the vector into cells is commercially available (for example, shRNA Cloning Vector from System Biosciences, LLC.). In addition, a reporter assay for analyzing a stress state and a drug response of cells using, as a marker, a transcription regulatory factor (for example, a p53 responsive element) that is linked to a reporter gene and activates in response to cellular stress such as DNA damage or oxidation is conventionally known (Non Patent Literature 6).

Non Patent Literature 7 and Patent Literature 1 describe a method for evaluating enzyme activity in which a variant of a gene encoding a known promoter or a known enzyme, and a vector containing a canvas sequence linked thereto are introduced into a cell, expression of a mutagenic protein is promoted in the cell by an enzyme expressed from the variant to induce a mutation in the canvas sequence, and the activity of the enzyme encoded by the variant is evaluated by the number of mutations in the canvas sequence. Non Patent Literature 8 describes a method for evaluating an action of a transcription factor on an enhancer by introducing gRNA that suppresses expression of a specific transcription factor and an enhancer linked to a barcode sequence into a cell and associating the gRNA with the enhancer activity based on the barcode sequence.

### Citation List

### Patent Literature

Patent Literature 1: WO 2023/028476

### Non Patent Literature

Non Patent Literature 1: Cell, 2013, 152(4): 909-922
Non Patent Literature 2: Nature biotechnology, 2016, 34(6): 634-636
Non Patent Literature 3: Cell, 2016, 167(7): 1853-1866
Non Patent Literature 4: Cell, 2016, 167(7): 1883-1896
Non Patent Literature 5: Nature methods, 2017, 14(3): 297-301
Non Patent Literature 6: Mutation Research, Genetic Toxicology and Environmental Mutagenesis, 2010, 696(1): 21-40.
Non Patent Literature 7: bioRxiv preprint, doi: doi.org/10.1101/2022.03.09.483646, Version 1, March 9, 2022
Non Patent Literature 8: bioRxiv preprint, doi: doi.org/10.1101/2020.09.30.321323, 2020

### Summary of the Invention

### Technical Problem

It is desirable to improve the efficiency and throughput of comprehensive analysis of functional molecules such as RNA and peptides.

### Solution to Problem

The present inventors have constructed a vector including a polynucleotide encoding an expression system of an RNA or peptide to be analyzed, and a polynucleotide encoding a transcriptional regulatory sequence that is activated by a response in a cell that can be caused by perturbation by the RNA or peptide and a reporter system operably linked thereto. A set of vectors in which each polynucleotide is arranged in a different vector is also available. When the vector or set of vectors is introduced into a cell, if the response occurs in the cell with perturbation by the expressed RNA or peptide, the reporter system is expressed accordingly. Therefore, the RNA or peptide can be associated with the response in the cell based on the expression of the reporter system in the cell, and thus the function thereof can be analyzed. Furthermore, it has been found that functions of a plurality of RNA or peptides can be analyzed at one time in one cell or cell population by introducing a plurality of vectors or sets of vectors including different RNAs or peptides or reporter systems into one cell or cell population and measuring expression of a reporter system derived from each vector.

Thus, the present invention provides the following.
[1] A vector or set of vectors for analyzing a function of a functional molecule, including
   a polynucleotide encoding an expression system of a candidate molecule of a functional molecule,
   a polynucleotide encoding a transcriptional regulatory sequence or translational regulatory sequence that is activated by a predetermined response in a cell, and
   a polynucleotide encoding a reporter system operably linked to the transcriptional regulatory sequence or translational regulatory sequence.
[2] The vector or set of vectors according to [1], in which the functional molecule is RNA or a peptide.
[3] The vector or set of vectors according to [1] or [2], in which the RNA is shRNA or guide RNA.
[4] The vector or set of vectors according to any one of [1] to [3], in which the transcriptional regulatory sequence or translational regulatory sequence that is activated by the predetermined response in the cell is a p53 binding sequence, an antioxidant responsive element, a nuclear factor kappa B binding sequence, an activating transcription factor 6 responsive sequence, a metal regulatory element, a heat shock element, a hypoxia responsive element, a xenobiotic responsive element, a nuclear factor of activated T-CELLS binding sequence, a TCF/LEF DNA binding protein binding sequence, sesRNA, and toehold RNA.
[5] The vector or set of vectors according to any one of [1] to [4], in which the transcriptional regulatory sequence activates a promoter arranged downstream thereof.
[6] The vector or set of vectors according to [5], in which the reporter system includes the promoter.
[7] The vector or set of vectors according to any one of [1] to [6], in which the reporter system is expressed in response to activation of the transcriptional regulatory sequence or translational regulatory sequence.
[8] The vector or set of vectors according to any one of [1] to [7], in which the reporter system is a luminescent or fluorescent protein reporter system, or an enzymatic reporter system.
[9] The vector or set of vectors according to [8], in which the enzymatic reporter system includes a combination of a protein having an effect of changing a target nucleotide sequence and its target sequence.
[10] The vector or set of vectors according to [8], in which the enzymatic reporter system expresses a DNA nuclease, a DNA recombinase, a transposase, or an integrase.
[11] The vector or set of vectors according to [8], in which the enzymatic reporter system includes a combination of an artificial nuclease and its recognition sequence, and a combination of a restriction enzyme and a corresponding restriction enzyme recognition sequence.
[12] The vector or set of vectors according to [8], in which the enzymatic reporter system includes a combination of a Cas family, guide RNA, and a Cas recognition sequence, a combination of a TALEN and a recognition sequence thereof, a combination of a ZFN and a recognition sequence thereof, or a combination of a base editor or prime editor and a recognition sequence thereof.
[13] The vector or set of vectors according to [11], in which the restriction enzyme is I-CeuI, I-SceI, PI-PspI, or PI-SceI.
[14] The vector or set of vectors according to [8], in which the enzymatic reporter system includes a CRE-LoxP system, a system having a sequence of a LoxP variant instead of or in addition to the LoxP sequence in the CRE-LoxP system, a Vika-Vox system, a Dre-rox system, or a Flp-FRT system.
[15] The vector or set of vectors according to any one of [1] to [14], in which the vector is a plasmid vector, a linear DNA sequence, a transposon vector, or a viral vector.
[16] The vector or set of vectors according to [15], in which the viral vector is a lentiviral vector, an adeno-associated viral vector, a baculoviral vector, an MMLV retroviral vector, or an MSCV retroviral vector.
[17] The vector or set of vectors according to any one of [1] to [16], further including a unique molecular identifier sequence.
[18] A method for searching a functional molecule causing a response in a cell, the method including
   introducing the vector or set of vectors according to any one of [1] to [17] into a cell, and
   measuring expression of a reporter system included in the vector or set of vectors in the cell.
[19] The method according to [18], in which the expression of the reporter system is quantified by luminescence or fluorescence measurement, protein quantification, quantitative RT-PCR, quantitative PCR, or sequencing.
[20] The method according to [18] or [19] further including
   adding two or more vectors or sets of vectors to a cell population to introduce each vector into any of the cells in the cell population, in which each of the two or more vectors or sets of vectors includes a polynucleotide encoding a different functional molecule.
[21] The method according to any one of [18] to [20], in which the cell is a cell collected from a human or a non-human animal, a cell derived from a specimen collected from a patient or a laboratory animal, a primary cultured cell, an organoid, a spheroid, an immortalized cultured cell, or a cell in a body of an individual of a laboratory animal.
[22] A method for searching a gene involved in a response of a cell, the method including
   introducing a plurality of vectors or sets of vectors into a cell population, in which each of the plurality of vectors or sets of vectors encodes
      RNA that interferes with a gene of interest,
      a transcriptional regulatory sequence or translational regulatory sequence that is activated by a predetermined response in a cell, and
      a reporter system operably linked to the transcriptional regulatory sequence or translational regulatory sequence,
   the RNA encoded by each of the plurality of vectors or sets of vectors being different from one another;
   measuring expression of a reporter system encoded by each of the plurality of vectors or sets of vectors in the cell populations, and
   determining whether the gene of interest is a gene involved in the predetermined response in the cell based on the expression of the reporter system included in each of the plurality of vectors or sets of vectors.
[23] The method according to [22], in which whether the gene of interest is a gene involved in the predetermined response in the cell is determined based on statistical value of the expression for the reporter system included in each of the plurality of vectors or sets of vectors.
[24] The method according to [22] or [23], in which the RNA that interferes with the gene of interest is shRNA or gRNA.
[25] A reagent kit including the vector or set of vectors according to any one of [1] to [17].
[26] The reagent kit according to [25], in which the kit is a diagnostic agent for a disease or a companion diagnostic agent.
[27] A method for diagnosing a disease, the method including implementing the method for searching a functional molecule causing a response in a cell according to any one of [18] to [21].
[28] A companion diagnostic method including implementing the method for searching a functional molecule causing a response in a cell according to any one of [18] to [21].
[29] Use of the vector or the set of vectors according to any one of [1] to [17] in search of a functional molecule that causes a response in a cell.
[30] Use of the vector or the set of vectors according to any one of [1] to [17] in search of a functional molecule that causes a response in a cell.
[31] Use of the vector or set of vectors according to any one of [1] to [17] in diagnosing a disease.
[32] Use of the vector or set of vectors described in any one of [1] to [17] in companion diagnostics.
[33] Use of the vector or the set of vectors according to any one of [1] to [17] in the manufacture of a diagnostic agent or a companion diagnostic agent for a disease.
[34] A method for analyzing an activity of a transcriptional regulatory sequence or translational regulatory sequence for a response in a cell, the method including:
   preparing a cell into which a vector has been introduced, in which
      the vector includes a polynucleotide encoding a transcriptional regulatory sequence or translational regulatory sequence, a polynucleotide encoding a reporter system operably linked to the transcriptional regulatory sequence or translational regulatory sequence, and a label sequence identifying the transcriptional regulatory sequence or translational regulatory sequence,
      the reporter system includes a protein having an effect of altering a target nucleotide sequence and a target sequence thereof, and expression of the reporter system alters the recognition sequence;
   in the cell, eliciting a predetermined response within a cell;
   after eliciting the response, analyzing the target sequence and the region including the label sequence in the vector and quantifying the alteration in the target sequence;
   associating the target sequence with the transcriptional regulatory sequence or translational regulatory sequence based on the label sequence; and
   evaluating the activity of the transcriptional regulatory sequence or translational regulatory sequence associated with the target sequence against the predetermined response in the cell based on the amount of alteration in the target sequence.
[35] The method according to [34], in which the vector includes a polynucleotide encoding the transcriptional regulatory sequence and the label sequence identifying the transcriptional regulatory sequence.
[36] The method according to [34] or [35], in which the region including the target sequence and the label sequence is analyzed by sequencing.
[37] The method according to any one of [34] to [36], the method further including
   introducing a plurality of vectors into a cell population, in which
   the plurality of vectors each include a polynucleotide encoding a different transcriptional regulatory sequence or translational regulatory sequence.

### Advantageous Effects of Invention

The present invention provides a vector or a vector useful for searching for a functional molecule that causes a response in a cell. The vector or vectors are designed such that a reporter system encoded by the same vector or set of vectors is expressed when a predetermined response is caused in a cell by a molecule such as RNA or peptide encoded by the vector. Therefore, according to the vector or the method for searching a functional molecule of the present invention using the vector, the molecule and the response can be associated based on the expression of the reporter system, and the function of the molecule can be analyzed. That is, the function of a molecule such as RNA or peptide can be estimated by a simple procedure. In addition, according to the present invention, since functions of a plurality of molecules can be analyzed at a time by one cell or cell population, the throughput of analysis is improved.

### Brief Description of Drawings

Fig. 1A illustrates structures of Plasmids 1 and 2.
Fig. 1B illustrates a structure of a plasmid 3.
Fig. 1C illustrates behavior of a reporter system in Plasmids 1 to 3.
Fig. 1D illustrates a structure of a plasmid 4.
Fig. 1E illustrates a structure of a linear DNA 1.
Fig. 1F illustrates a structure of a plasmid 5.
Fig. 2 illustrates fluorescence expression by doxorubicin treatment in Plasmid 1-introduced cells. Vertical axis: Red/green fluorescent positive cells, horizontal axis: doxorubicin concentration and time from doxorubicin concentration. Each dot represents a value obtained by dividing the number of red fluorescent positive cells obtained from each culture well by the number of green fluorescent positive cells. Bars represent mean values.
Fig. 3 illustrates an inversion rate (RQ) of mKate2 sequence in Plasmid 1 after doxorubicin treatment.
Fig. 4 illustrates a ratio of red/green fluorescence positive cells among Plasmids 1 and 2 introduced cells after doxorubicin treatment. DMSO: No addition of doxorubicin, Doxo: Addition of doxorubicin. shCon: sh-scramble (Plasmid 1) introduced cell, shRAD51: shRAD51 (Plasmid 2) introduced cell. Each dot represents a value obtained by dividing the number of red fluorescent positive cells obtained from each culture well by the number of green fluorescent positive cells. Bars represent mean values.
Fig. 5 illustrates an inversion rate of the mKate2 sequence in the plasmid 3 after TBHQ treatment. Flip: Inverted arrangement ratio, Original: Non-inverted arrangement ratio. The horizontal axis represents the TBHQ concentration.
Fig. 6 illustrates activation of a Wnt pathway in a cell at 6 hours and 24 hours after CHIR99021 treatment. Top: AXIN2 expression, bottom: SP5 expression. The expression level is a relative amount with respect to CHIR996021 untreated (0 µM). The horizontal axis represents the CHIR99021 concentration.
Fig. 7 illustrates the amount of marker sequence inversion in the vector after CHIR99021 treatment. Linear: Linear DNA1, Plasmid: plasmid 4. The horizontal axis represents the CHIR99021 concentration. The expression level is a relative amount with respect to CHIR99021 untreated (0 µM).
Fig. 8 illustrates the inversion amount of the mKate2 sequence induced by shRNA in viral vector-introduced cells. Scramble: Viral vector 1A introduced cell, shRAD51: Viral vector 1B introduced cell. Top: Expression level of MOI 0.4 in cells, bottom: expression level of MOI 2 in cells.
Fig. 9 is a schematic diagram of amplicons used for quantification of inverted DNA sequences in Example 7. shCon: Amplicon including sh-scramble, shRad_2, and shRad_4: amplicon including shRAD51_2 and shRAD51_4.
Fig. 10 illustrates the inversion rate (inversion/non-inversion) of the mKate2 sequence in each plasmid in plasmid pool introduced cells. Left: No-addition of doxorubicin, and right: addition of 1µM of doxorubicin. The number on the horizontal axis is the well number. shControl: plasmid including sh-scramble, shRad51_2 and shRad51_4: plasmid including shRAD51_2 and shRAD51_4.
Fig. 11A illustrates inversion odds of a marker sequence on each vector in DNA derived from cells into which shRNA-pooled vector linear DNA 2 has been introduced. Scramble: sh-scramble cells, Targeting: gene target shRNA introduced cells. The horizontal axis represents the gene targeted by the expressed shRNA and the ID number of the shRNA targeting each gene.
Fig. 11B illustrates results of averaging the inversion odds of the marker sequence on each vector in DNA derived from cells into which linear DNA 2 was introduced, for each target gene of shRNA. The horizontal axis represents a gene targeted by the shRNA. The plot in the graph illustrates the inversion odds of marker sequences on the same vector as each shRNA.
Fig. 12 illustrates inversion and deletion odds of marker sequences on each vector in DNA derived from cells into which shRNA-pooled vector linear DNA3 was introduced. The horizontal axis represents the gene targeted by the expressed shRNA and the ID number of the shRNA targeting each gene.
Fig. 13 illustrates a structure of a linear DNA 4.
Fig. 14A illustrates a preparation procedure of a linear DNA 4A.
Fig. 14B illustrates a preparation procedure of a linear DNA 4B.
Fig. 15 illustrates a structure of a plasmid 6.
Fig. 16 illustrates inversion odds of a marker sequence on each vector in DNA derived from cells into which shRNA-pooled vector linear DNA 4 has been introduced. The horizontal axis represents the gene targeted by the expressed shRNA and the ID number of the shRNA targeting each gene. Left: No filtering by UMI, right: filtering by UMI.
Fig. 17 illustrates inversion odds of mKate2 sequence in minP variant sequence-introduced cells. Original: Original minP, Variant: minP variant.

### Description of Embodiments

All patent, non-patent, and other publications cited herein are incorporated herein by reference in their entirety.

Each component exemplified in the present specification can be used alone or in combination of two or more unless otherwise specified.

In the present specification, the notation representing a numerical range such as "A to B" is synonymous with "A or more and B or less", and A and B are included in the numerical range.

The protein exemplified in the present specification includes a mutant protein in which the amino acid sequence or the base sequence encoding the amino acid sequence is substituted, overlapped, deleted, inserted, or frameshifted, when the mutant protein has a function equivalent to that before mutation.

In the present specification, the "operable linkage" between a sequence encoding RNA, a peptide, a reporter, or the like and a regulatory sequence including a transcriptional regulatory sequence such as a promoter and a translational regulatory sequence means that the RNA, the peptide, the reporter, or the like is functionally linked so as to be capable of being expressed under the regulation of the regulatory sequence. The procedure of "operable linkage" of an expressed sequence to a regulatory sequence is well known to those skilled in the art. For example, "the reporter is operably linked to a transcriptional regulatory sequence" means that the reporter is transcribed under the regulation of the transcriptional regulatory sequence, and "the translational regulatory sequence and the reporter are operably linked" means that the reporter is translated under the control of the translational regulatory sequence. Therefore, in the present specification, the "operable linkage" between the coding sequence and the regulatory sequence includes not only a case where the coding sequence is subjected to expression control in a regulatory sequence present in the same nucleotide chain, but also a case where the coding sequence and a regulatory sequence that controls expression thereof are present in separate nucleotide chains.

In the present specification, the "expression" of a gene or an expression product thereof includes transcription of a gene and translation of a transcript. For example, the term "expression" includes transcription of a gene into mRNA, translation of mRNA into protein, and expression of the protein itself and the mRNA encoding it.

In the present specification, the terms "upstream" and "downstream" with respect to a nucleotide sequence mean regions on the 5' side and the 3' side, respectively, in a DNA sense strand of the nucleotide sequence.

In the present specification, the term "perturbation" in a cell refers to knockdown, knockout, or expression activation of a target (for example, a gene or mRNA thereof) induced by expression of RNA complementary to the target, or expression in a cell of a functional molecule such as a peptide. Examples of RNA that induces perturbation include shRNA and gRNA.

In the present specification, the term "response" in the cell represents an event in the cell caused by perturbation, and examples thereof include stress such as DNA damage or oxidation in the cell caused by expression of the RNA or peptide, a change in the expression level or activity of a given gene or a change in the intracellular environment due to the change, and the like.

A "functional molecule" herein refers to a molecule that induces a perturbation in a cell. In a case where the perturbation causes a response in a cell, the functional molecule causes an intracellular response.

The "polynucleotide" herein may be single-stranded DNA, double-stranded DNA, single-stranded RNA, double-stranded RNA, or other artificial nucleic acids as long as it can retain information of the RNA or peptide it encodes. Among them, double-stranded DNA is preferable from the viewpoint of being able to stably store information.

### (Method for searching functional molecule causing Response in Cell)

The present invention provides a method for searching a functional molecule causing a response in a cell. Examples of functional molecules (hereinafter, also referred to as "target molecule") that are targets to be searched for by the present method and cause a response in a cell include small hairpin RNA (shRNA), guide RNA (gRNA), enhancer RNA, small activating RNA, RNA such as long non-coding RNA, and peptides.

The shRNA to be searched for in the method of the present invention is RNA having a stem loop structure, the stem loop structure consisting of 40 to 100 nucleotides, the stem portion consisting of 19 to 45 nucleotides, and the loop portion consisting of 4 to 25 nucleotides. The shRNA to be searched for in the method of the present invention is RNA having a stem loop structure, the stem loop structure consisting of 45 to 75 nucleotides, the stem portion consisting of 19 to 30 nucleotides, and the loop portion consisting of 6 to 15 nucleotides. The sequence of the stem portion of the shRNA has complementarity to and may bind to a nucleotide sequence in a cell (mRNA, genomic DNA, genomic RNA, or the like).

The guide RNA (gRNA) to be searched for in the method of the present invention is RNA having a sequence of 10 to 250 bases complementary to the target sequence.

The enhancerRNA searched for in the method of the present invention is 15 to 2,000 nucleotides in length.

The small activating RNA to be searched for in the method of the present invention is RNA having a complementary sequence of 14 to 30 nucleotides between 2,000 bp upstream of the transcription start point of the target gene and 2,000 bp downstream of the transcription start point.

The long non-coding RNA to be searched for in the method of the present invention is RNA sequence having no coding region of 200 nucleotides or more.

The peptide to be searched for in the method of the present invention is not particularly limited in its molecular size, and examples thereof include proteins, poly or oligopeptides. The target peptide to be searched for in the method of the present invention is, for example, a peptide that functions as a signal factor, an enzyme, a coenzyme, or the like in a cell and can cause a perturbation in the cell.

The method for searching a functional molecule according to the present invention basically includes screening molecules such as RNA and peptide that are candidates for the target molecule. Among them, functional molecules that trigger specific responses to be targeted in the cell are determined. The method is characterized using a vector or a set of vectors provided by the present invention, in which the vector or set of vectors includes a polynucleotide encoding an expression system of a functional molecule (hereinafter, also referred to as "candidate molecule") that is a candidate for the target molecule (that is**,** of unknown relevance to an intracellular response), a polynucleotide encoding a regulatory sequence (transcriptional regulatory sequence or translational regulatory sequence) that can be caused by the target molecule (that is**,** to investigate the relationship with the candidate molecule) and is activated by a predetermined response in a cell, and a polynucleotide encoding a reporter system operably linked to the regulatory sequence.

In the present invention, a polynucleotide encoding an expression system of the candidate molecule, a polynucleotide encoding a regulatory sequence that is activated by the predetermined response in the cell, and a polynucleotide encoding a reporter system operably linked to the regulatory sequence may be arranged on the same vector, or may be arranged on different vectors. The arrangement of the polynucleotide encoding the expression system of the candidate molecule, the polynucleotide encoding the regulatory sequence activated by the predetermined response in the cell, or the polynucleotide encoding the reporter system on each vector is not particularly limited as long as the candidate molecule is expressed, the regulatory sequence is activated by the response in the cell, and the expression of the reporter system is promoted by the activation of the regulatory sequence.

In one embodiment, there is provided one vector in which a polynucleotide encoding an expression system of the candidate molecule and a polynucleotide encoding the regulatory sequence and a polynucleotide encoding the reporter system arranged in order from upstream are arranged. In another embodiment, there is provided a vector set including a vector in which a polynucleotide encoding an expression system for the candidate molecule is arranged, and a vector in which a polynucleotide encoding the regulatory sequence and a polynucleotide encoding the reporter system are arranged in this order from upstream. In still another embodiment, there is provided a vector set including a vector in which a polynucleotide encoding an expression system of the candidate molecule and a polynucleotide encoding a part of the reporter system (for example, an enzyme recognition sequence, LoxP or a variant thereof and a marker sequence described later, or a sequence transferred by a transposase or an integrase) are arranged, and a vector in which a polynucleotide encoding the regulatory sequence and a polynucleotide encoding the remaining part of the reporter system are arranged in order from the upstream.

In one embodiment, the polynucleotide encoding the expression system of the candidate molecule includes a polynucleotide encoding an RNA expression system. For example, the expression system includes a sequence encoding a double-stranded RNA (for example, shRNA) including an RNA sequence that is the candidate molecule, and a transcriptional regulatory sequence such as an RNA Pol III promoter (for example, U6 promoter, H1 promoter, and the like) or an RNA Pol II promoter (such as a CMV promoter) operably linked to the double-stranded RNA. In a case where the candidate molecule is a gRNA, a protein (for example, Cas9 protein, Cas9 nickcase, dCas9/VP64, dCas9/VPR, dCas9/KRAB, dCas9/KRAB/MeCP2, Base editor, or the like) for causing the gRNA to function may be co-expressed. In another embodiment, the polynucleotide encoding the expression system of the candidate molecule encodes an expression system of a peptide including a sequence encoding the peptide as the candidate molecule and a transcriptional regulatory sequence such as a promoter operably linked thereto. Vectors including the nucleotide sequences encoding the RNA of interest or the peptide of interest and their expression systems can be synthesized and purchased through commercial services.

Regulatory sequences that are activated by the predetermined response in the cell include transcriptional and translational regulatory sequences. Examples of the transcriptional regulatory sequence include a transcriptional regulatory sequence that is directly or indirectly activated by a response in a cell to be examined whether the transcriptional regulatory sequence is caused by the candidate molecule, for example, a DNA damage response, an oxidative stress response, or protein expression, for example, signaling pathway activation such as Wnt pathway. More specific examples of such transcriptional regulatory sequences include a p53 binding sequence (p53RE), an antioxidant responsive element (ARE), a nuclear factor kappa B (NF-kB) binding sequence, an activating transcription factor 6 responsive element (ATF6 ERSE), a metal regulatory element (MRE), a heat shock element (HSE), a hypoxia responsive element (HRE), a xenobiotic responsive element (XRE), a nuclear factor of activated T-CELLS (NFAT) binding sequence, and a TCF/LEF type DNA binding protein binding sequence. These transcriptional regulatory sequences activate a promoter arranged downstream thereof, which promotes expression of the reporter system described below. The type of the promoter is not limited as long as the promoter can be activated under the regulation of the transcriptional regulatory sequence. The promoter may be included in a polynucleotide encoding the transcriptional regulatory sequence, or may be contained in a reporter system described later.

The translational regulatory sequence that is activated by the predetermined response in the cell is, for example, a sequence that is activated according to the response in the cell to be examined whether the translational regulatory sequence is caused by the candidate molecule and promotes the translation of the downstream coding RNA. More specific examples of the translational regulatory sequence include sesRNA. The sesRNA is a module that expresses downstream coding RNA by any mRNA molecule and the activity of the ADAR protein in the cell, and is a sensor of mRNA expression (see Nature, 2022,610:713-721, Nature Biotechnology, 2023, 41:698-707, Nature Biotechnology, 2023, 41:482-487, WO/2023/077135, WO/2023/077138, US20230123513). The sesRNA includes an RNA sequence complementary to a specific target mRNA. When the ADAR protein in a cell acts on the double strand of the sesRNA and the mRNA molecule, the coding RNA linked downstream becomes translatable. If a sequence encoding the reporter system is arranged downstream of the sesRNA coding sequence, the expression of the reporter system reflects the expression of the target mRNA.

Another specific example of the translational regulatory sequence includes toehold RNA. The toehold RNA includes a sequence complementary to the target mRNA, a ribosome binding sequence, and is linked to a downstream coding RNA. Since the toehold RNA normally forms a hairpin loop, the coding RNA is not translated, but when bound to the target mRNA, the hairpin disappears and is translated (see Toehold switch, Nature Biotechnology, 2022, 40:53-545). If a sequence encoding the reporter system is arranged downstream of the toehold RNA coding sequence, the expression of the reporter system reflects the expression of the target mRNA.

The reporter system encoded by the vector provided in the present invention is operably linked to the regulatory sequence and is expressed in response to activation of the regulatory sequence. Thus, expression of the reporter system represents that the candidate molecule has caused a predetermined response in a cell. By measuring the expression of the reporter system, it can be determined whether the candidate molecule is one that triggers the predetermined response in the cell (that is, whether the target molecule is a target molecule for search).

Examples of the reporter system include a luminescent or fluorescent protein reporter system and an enzymatic reporter system. The luminescent or fluorescent protein reporter system expresses a luminescent or fluorescent protein. The expressed luminescent or fluorescent protein is detected as a marker. The enzymatic reporter system expresses an enzyme that produces a detectable marker molecule. A molecule generated by the function of the expressed enzyme is detected as a marker. Examples of enzymes expressed by the enzymatic reporter system include DNA nucleases, DNA recombinases, hydrolases (for example, β-glucuronidase), transposases, integrases, and the like. Among them, a protein having an action of changing the target nucleotide sequence (DNA nucleases, DNA recombinases, transposases, integrases, or the like) is preferable. Therefore, an example of the enzymatic reporter system includes a combination of a protein having an action of changing the target nucleotide sequence and its target sequence.

Examples of the DNA nuclease include Cas family such as Cas9 nuclease, artificial nucleases such as transcription activator-like effector nuclease (TALEN), and Zinc Finger Nuclease (ZFN), Base Editor, Prime Editor, and the like; restriction enzymes, or the like. The restriction enzyme preferably forms a sticky end at the cleavage site, and examples thereof include I-CeuI, I-SceI, PI-PspI, and PI-SceI.

Examples of the enzymatic reporter system using an artificial nuclease include a combination of an artificial nuclease and a recognition sequence thereof, for example, a combination of a Cas family such as a Cas9 nuclease, a guide RNA (gRNA or sgRNA), and a Cas recognition sequence, a combination of Base Editor or Prime Editor and a recognition sequence thereof by modifying a nucleic acid recognition protein of the artificial nuclease described above, a combination of a TALEN and a recognition sequence thereof, or a combination of a ZFN and a recognition sequence thereof. Preferably, the recognition sequence of the TALEN or ZFN consists of 30 to 50 nucleotides. Examples of enzymatic reporter systems using restriction enzymes include combinations of restriction enzymes and corresponding restriction enzyme recognition sequences. In these systems, a DNA sequence cleaved by an artificial nuclease or a restriction enzyme can be detected as a marker. The recognition sequence of the artificial nuclease or the restriction enzyme recognition sequence may be arranged on the same vector as or different from the vector containing the polynucleotide encoding the artificial nuclease or the restriction enzyme, or may be arranged on a different vector. For example, the recognition sequence may be arranged on a vector containing a polynucleotide encoding an expression system of the candidate molecule. Examples of means for detecting a cleaved DNA sequence include next generation sequencing such as massively parallel sequencing, PCR, and the like.

Examples of enzymatic reporter systems using the DNA recombinase include the CRE-LoxP system. The system is composed of a CRE recombinase and two LoxP sequences and a marker sequence arranged therebetween. The LoxP sequence and the marker sequence may be arranged on the same vector as that containing the polynucleotide encoding the CRE recombinase, or may be arranged on different vectors. For example, the LoxP sequence and marker sequence may be arranged on a vector containing a polynucleotide encoding an expression system for the candidate molecule. The two LoxP sequences may be arranged in series in the same direction, or may be arranged in series in opposite directions. In a case where the LoxP sequences are arranged in the same direction, the marker sequence in between is cut out by the action of CRE, whereas in a case where the LoxP sequences are arranged in opposite directions, the marker sequence in between is inverted by the action of CRE. Therefore, the cut-out sequence itself may be detected as a marker sequence, a sequence generated on the vector by cutting out the sequence may be detected as a marker sequence, or an inverted marker sequence may be detected. Examples of means for detecting the Maker sequence include next generation sequencing such as massively parallel sequencing, PCR, and the like.

Alternatively, the marker sequence is arranged in a direction opposite to the transcription direction between two LoxP sequences arranged in the opposite direction, and a promoter is linked upstream of the LoxP sequence on the vector. In a state in which CRE does not act, the marker is not expressed, but when the marker sequence is inverted between LoxP sequences by the action of CRE, that is, rearranged in a transcribable direction, the marker is expressed by the action of the promoter. The expressed marker may be detected. In this case, a sequence encoding a luminescent or fluorescent protein is preferable as the marker sequence because it is easily detected. Examples of preferred combinations of promoter and marker sequence include combinations of EF1 promoter and mKate2 coding sequence.

Examples of reporter systems having effects similar to the CRE-LoxP system described above include systems having sequences of LoxP variants instead of or in addition to the LoxP sequence in the CRE-LoxP system, the Vika-Vox system, the Dre-Rox system, and the Flp-FRT system. LoxP variants include Lox71, Lox66, Lox5171, Lox2722, and Loxm2. In the present specification, the CRE-LoxP system and the reporter system having an action similar thereto are also collectively referred to as a "CRE-LoxP-like system".

An example of the enzymatic reporter system using the transposase is the piggyBac transposon system. The system includes piggyBac transposase and two inverted terminal repeat sequences (ITR). The two ITRs are arranged in series in opposite directions, and an arbitrary marker sequence is arranged therebetween. The ITR and the marker sequence may be arranged on the same vector as that containing the polynucleotide encoding the transposases, or may be arranged on different vectors. For example, the ITR and marker sequence may be arranged on a vector containing a polynucleotide encoding an expression system for the candidate molecule. Transfer of the marker sequence between the two ITRs by the action of the transposase (disappearance of the marker sequence from between the ITRs) may be detected. Alternatively, the TTAA sequence may be provided in the vector, and a marker sequence transferred to the position of the TTAA sequence may be detected. Alternatively, a marker sequence transferred to the TTAA sequence on the genome may be detected. In one embodiment, a polynucleotide encoding an expression system of the candidate molecule described above may be arranged as the marker sequence, and disappearance of the polynucleotide from between ITRs or transfer of the polynucleotide to the position of the TTAA sequence may be detected. Examples of means for detecting the Maker sequence include next generation sequencing such as massively parallel sequencing, PCR, and the like.

An example of the enzymatic reporter system using the integrase is the phiC31/attP system. The system includes a vector including the phiC31 integrase and a donor vector including the attB site. The phiC31 integrase recombines the attP site of the host genome with the attB site of the donor vector, and incorporates the donor vector into the genome. The sequence of the donor vector may be used as a marker, and the marker sequence transferred to the genome may be detected. Examples of means for detecting the Maker sequence include next generation sequencing such as massively parallel sequencing, PCR, and the like.

The vector or set of vectors used in the present invention may further include a unique molecular identifier (UMI) sequence. For example, an individual vector molecule is identified by a unique UMI, regardless of the encoding candidate molecule, regulatory sequence, or reporter system. By performing analysis in consideration of UMIs, it is possible to eliminate the influence of sequence amplification (PCR during cell division or library production or the like) occurring in the process of analysis, and to reduce bias. For example, it is possible to analyze only the expression level of a reporter system to which a unique UMI is given, or to correct the expression level of a reporter associated with the UMI based on the number of detected UMIs.

The type of the vector provided in the present invention is not particularly limited as long as it can express the held candidate molecule or reporter system in a host cell into which the vector is introduced. Examples of the vector include a plasmid vector, a linear DNA sequence, a transposon vector, and a viral vector. The viral vector may be a DNA vector or an RNA vector, and examples include lentiviral vectors, adeno-associated viral vectors, baculoviral vectors, MMLV retroviral vectors, and murine stem cell virus (MSCV) retroviral vectors. The vector may exist outside the nucleus in the introduced cell, or may exist by being incorporated into the genome. The incorporation of the polynucleotide into the vector can be implemented according to a conventional method.

In the method for searching a functional molecule according to the present invention, the vector or the set of vectors is introduced into a cell, and then the expression of the reporter system included in the vector or the set of vectors in the cell is measured.

The cell (host cell) into which the vector is introduced may be a mammalian cell or a non-mammalian cell. Preferably, the cell is a cell of a mammal such as a human, a mouse, or a rat. Also preferably, the cell is a microbial cell. Preferably, the cell is a cultured cell. Examples of the cell include a cell collected from a human or a non-human animal (for example, mice, rats, and the like), a cell derived from a specimen collected from a patient or a laboratory animal, a primary cultured cell, an organoid, a spheroid, an immortalized cultured cell, a cell in a tissue slice derived from a specimen, and a microbial cell such as E. coli. Cells in the body of an individual laboratory animal can also be used.

The introduction of the vector into the host cell can be implemented according to a conventional method such as a calcium phosphate method, an electroporation method, a lipofection method, a particle gun method, or a PEG method. Reagents and devices for vector introduction into cells are commercially available.

The means for measuring the expression of the reporter system can be appropriately selected according to the type of the reporter system. Means capable of quantifying a marker generated from the reporter system is preferable. In one example, in a case where the marker is a protein, examples thereof include known protein quantification methods such as colorimetry, absorbance measurement, chromatography, or immunoassay, and methods for quantifying mRNA encoding a protein such as quantitative RT-PCR, or sequencing. In another example, in a case where the marker is a luminescent or fluorescent protein, examples of the measurement means include luminescence or fluorescence measurement methods such as luminescence or fluorescence imaging, luminescence or fluorescence photometry.

In another example, in a case where the marker generated from the reporter system is a DNA sequence cleaved by an artificial nuclease or a restriction enzyme, a mutation of a DNA sequence induced by cleavage by an artificial nuclease or a restriction enzyme (substitution or deletion of a base generated in a cleavage site at the time of cleavage), or a DNA sequence mutated by a protein that mutates a recognition sequence such as Base Editor or Prime Editor, examples of means for measuring expression of the reporter system include a method capable of detecting an abundance of the cleaved sequence or the mutated DNA sequence such as next generation sequencing such as quantitative PCR or massively parallel sequencing. For example, in a case where a recognition sequence of an artificial nuclease or a restriction enzyme recognition sequence is arranged on the vector, the expression level of the reporter system can be quantified by amplifying and quantifying a part of the vector sequence including the cleavage site by PCR or quantifying the sequence reads using the part of the vector sequence including the cleavage site as a reference sequence.

In another example, in a case where the reporter system is the CRE-LoxP system described above, the measurement means includes a method of quantifying a marker sequence cut out from between LoxP sequences arranged in the same direction, a method of quantifying a vector sequence remaining after cutting out the sequence, and a method of quantifying an inverted marker sequence between LoxP sequences arranged in opposite directions. For quantification of the sequence, quantitative PCR, next generation sequencing such as massively parallel sequencing, or the like can be used. Alternatively, when the marker sequence encoding the protein is linked in the opposite direction between the LoxP sequences arranged in the opposite direction as described above, the marker protein expressed from the marker sequence inverted by CRE can be quantified by the protein quantification method or the luminescence or fluorescence measurement method described above. The same applies to other CRE-LoxP-like systems.

In another example, in a case where the reporter system is a system using the above-described transposase or integrase, the measurement means includes a method of quantifying a transferred marker sequence or a sequence in which a marker sequence is lost. For quantification of the sequence, quantitative PCR, next generation sequencing such as massively parallel sequencing, or the like can be used.

Expression of the reporter system indicates that the candidate molecule has produced a predetermined response in a given cell by activating the regulatory sequence included in the vector or set of vectors. By measuring the expression of the reporter system, it can be determined whether the candidate molecule is one that triggers the predetermined response in the cell (that is, whether it is a target molecule for the search).

In one embodiment, the method of the present invention includes introducing into a cell at least one vector or set of vectors and measuring expression of the reporter system from the vector or set of vectors.

In the present specification, "introducing at least one type of vector into one cell" means introducing at least one type of common vector into at least one cell, and includes the case of introducing one type or two or more types of vectors into a single cell, the case of introducing at least one type or two or more types of common vectors into a plurality of cells, and the case of introducing different types of vectors into each of a plurality of cells. In either case, the total number of vectors introduced per cell may be one or two or more. Each of the introduced vectors includes a polynucleotide encoding an expression system for the candidate molecule, a polynucleotide encoding the regulatory sequence, and a polynucleotide encoding the reporter system.

In addition, in the present specification, "introducing at least one set of vectors into one cell" means introducing at least one type of common set of vectors into at least one cell, and includes the case of introducing one type or two or more types of set of vectors into a single cell, the case of introducing at least one type or two or more types of common set of vectors into a plurality of cells, and the case of introducing different types of sets of vectors into each of a plurality of cells. In either case, the total number of sets of vectors introduced per cell may be one or two or more. The introduced set of vectors includes a polynucleotide encoding an expression system of the candidate molecule, a polynucleotide encoding the regulatory sequence, and a polynucleotide encoding the reporter system, and these polynucleotides are included in any of two or more vectors constituting the set of vectors.

Preferably, in the method of the present invention, the response in a cell caused by the candidate molecule included in each of the two or more vectors or the set of two or more vectors is analyzed. For example, in the method of the present invention, two or more vectors or a set of two or more vectors are added to one cell population, each vector or set of vectors is introduced into any cell in the cell population, and then expression of the reporter system from each vector or set of vectors is measured.

The configuration and procedure of the method of the present invention in a case where two or more types of vectors (each of which includes a polynucleotide encoding an expression system for the candidate molecule, a polynucleotide encoding the regulatory sequence, and a polynucleotide encoding the reporter system) are used are described below. It is clear for those skilled in the art that the method of the present invention can be implemented with the same configuration and procedure even in a case where a set of two or more types of vectors (each set including a polynucleotide encoding an expression system for the candidate molecule, a polynucleotide encoding the regulatory sequence, and a polynucleotide encoding the reporter system) is used instead of the two or more types of vectors.

In one embodiment, each of the two or more types of vectors includes a polynucleotide encoding a respective different regulatory sequence, each of which is activated by a response in different cells, and a polynucleotide encoding a respective different reporter system operably linked to the regulatory sequence, while the candidate molecules encoded by each vector may be the same or different from each other, preferably different for each vector. Preferably, the reporter system is a luminescent or fluorescent protein reporter system.

In another embodiment, the reporter system is a combination of an artificial nuclease and its recognition sequence, or a combination of a restriction enzyme and a corresponding restriction enzyme recognition sequence.

In one example, the two or more types of vectors each include a polynucleotide encoding the same or different candidate molecules, a polynucleotide encoding a different regulatory sequence that is activated by a response in different cells, and a polynucleotide encoding a different reporter system operably linked to the regulatory sequence. In another example, the two or more types of vectors encode a common candidate molecule, while each encodes a different regulatory sequence (each activated by a response in different cells) and a sequence of a reporter system. In another example, the two or more vectors each encode a different candidate molecule while encoding common regulatory sequences and sequences for a reporter system.

In the present embodiment, a vector cleaved by an enzyme is generated according to a response in a cell. The vector fragments cleaved due to the action of each candidate molecule are then quantified. In a case where quantification is performed by PCR, two types of reverse primers are used which bind to the upstream and downstream of the estimated cleavage site (enzyme recognition sequence) of the vector, respectively. As the forward primer, a primer that binds to a sequence encoding each candidate molecule can be used. Alternatively, the forward primer may be a primer that binds to an identifier sequence (for example, a different sequence for each shRNA adjacent to the shRNA coding sequence) corresponding to the sequence encoding each candidate molecule. When the value obtained by dividing the amount of the amplification product by the reverse primer binding to the upstream of the estimated cleavage site by the amount of the amplification product by the reverse primer binding to the downstream is higher than the value in the control template in which no cleavage has occurred, it is considered that cleavage has occurred in the vector due to the presence of the candidate molecule. Detecting cleavage of the vector indicates that each candidate molecule has caused a response in a cell.

In another embodiment, the reporter system of the vector is a combination of Base Editor or Prime Editor and its recognition sequence.

In one example, the two or more types of vectors each include a polynucleotide encoding the same or different candidate molecules, a polynucleotide encoding a different regulatory sequence that is activated by a response in different cells, and a polynucleotide encoding a different reporter system operably linked to the regulatory sequence. In another example, the two or more types of vectors encode a common candidate molecule, while each encodes a different regulatory sequence (each activated by a response in different cells) and a sequence of a reporter system. In another example, the two or more vectors each encode a different candidate molecule while encoding common regulatory sequences and sequences for a reporter system.

In the present embodiment, a mutation is introduced into the sequence on the vector according to the response in the cell. The vector fragments mutated due to the action of each candidate molecule are then quantified. In a case where quantification is performed by PCR, as described above, vector fragments containing each candidate molecular sequence or a label sequence associated therewith and mutated sequences are amplified and quantified by PCR. In PCR, a forward primer that binds to the same candidate molecular sequence as described above or a label sequence associated therewith, and a reverse primer that links to the mutated sequence can be used. Detection of an amplification product indicates that the respective candidate molecule elicited a response in the cell. In a case where quantification is performed by sequencing, a sequence of a vector fragment including each candidate molecular sequence or the label sequence and a mutated sequence is used as a reference sequence. The mutated sequence may be, for example, a sequence targeted by a protein having an action of changing the target nucleotide sequence described above and a surrounding sequence thereof. The various vectors in the cell are collectively subjected to sequencing, and reads corresponding to each reference sequence are detected or quantified. In a case where there is a read corresponding to the reference sequence, for example, in a case where there is a sequence alteration in the target sequence or its peripheral sequence region in the mapped read, it indicates that each candidate molecule has caused a response in a cell.

In another embodiment, the reporter system includes a CRE-LoxP-like system.

In one example, the two or more types of vectors each include a polynucleotide encoding the same or different candidate molecules, a polynucleotide encoding a different regulatory sequence that is activated by a response in different cells, and a polynucleotide encoding a different reporter system operably linked to the regulatory sequence. In another example, the two or more types of vectors encode a common candidate molecule, while each encodes a different regulatory sequence (each activated by a response in different cells) and a sequence of a reporter system. The CRE-LoxP-like system is selected from the group consisting of a CRE-LoxP (and/or LoxP variant) system, a Vika-Vox system, a Dre-Rox system, and a Flp-FRT system, and a different type of CRE-LoxP-like system is used for each vector.

In another example, the two or more types of vectors each encode a different candidate molecule, while encoding a common regulatory sequence and sequence of a reporter system (CRE-LoxP-like system).

The two LoxP-like sequences (a sequence of two LoxPs or variants thereof, two Vox sequences, two Rox sequences, or two FRT sequences, including a marker sequence) of the reporter system may be arranged on the vector encoding the candidate molecule, in which different types of LoxP-like moieties may be arranged on the same vector. For example, the two LoxP-like sequences are arranged in opposite directions to each other, and a marker sequence is arranged therebetween. The type of marker sequence may be the same or different for each vector.

In the present embodiment, the marker sequence between LoxPs is inverted according to the response in the cell. Next, a vector fragment including a sequence encoding each candidate molecule and an inverted marker sequence is quantified. In a case where quantification is performed by PCR, each candidate molecular sequence or a vector fragment including a label sequence associated therewith and the inverted marker sequence is amplified and quantified by PCR in the same manner as described above. PCR can use a forward primer that binds to each candidate molecule sequence or its associated label sequence as described above, and a reverse primer that binds to the inverted marker sequence. The presence of an amplification product indicates that the respective candidate molecule elicited a response in the cell. In another example, the vector sequence obtained from the cell is amplified using a forward primer that anneals with each candidate molecular sequence or a label sequence associated therewith and a reverse primer designed to increase the sequence including the marker sequence. The inversion amount of the marker sequence in the amplification product can be quantified by performing quantitative PCR. When quantification is performed by sequencing, the sequence of a vector fragment including a sequence encoding a candidate molecule and a marker sequence is used as a reference sequence. At this time, a series of reference sequences covering each candidate molecular sequence or a combination of the label sequence and the inversion marker sequence is prepared. The various vectors in the cell are collectively subjected to sequencing, and reads corresponding to each reference sequence are detected or quantified. The presence of a read corresponding to each reference sequence, for example, the presence of a read corresponding to each reference sequence in which the marker sequence has altered from the original, indicates that each candidate molecule has caused a response in a cell.

In another embodiment, the reporter system includes a piggyBac transposon system or a phiC31/attP system.

In one example, the two or more types of vectors each include a polynucleotide encoding the same or different candidate molecules, a polynucleotide encoding a different regulatory sequence that is activated by a response in different cells, and a polynucleotide encoding a different reporter system operably linked to the regulatory sequence. In another example, the two or more types of vectors encode a common candidate molecule, while each encodes a different regulatory sequence (each activated by a response in different cells) and a sequence of a reporter system. The type of marker sequence transferred by the reporter system is different for each vector.

In another example, the two or more vectors each encode a different candidate molecule while encoding common regulatory sequences and sequences for a reporter system.

In the present embodiment, a given marker sequence is transferred in response to a response in a cell. Next, the sequence encoding each candidate molecule and the transferred marker sequence or the vector sequence remaining after the transfer of the marker sequence are quantified. Preferably, the vector is constructed in advance so that the sequence of the vector including the candidate molecule or the label sequence associated with the candidate molecule is altered by the transfer of the marker sequence, and then the sequence encoding the candidate molecule and the presence or absence of the sequence alteration caused by the transfer of the marker sequence in the sequence are quantified.

In quantification, for example, in a case where quantification is performed by PCR, each candidate molecular sequence and marker sequence are amplified. The presence of an amplification product indicates that the respective candidate molecule elicited a response in the cell. In a case where quantification is performed by sequencing, a sequence including each candidate molecular sequence or marker sequence is used as a reference sequence, and a read corresponding to each reference sequence is detected or quantified. The presence of a read corresponding to each reference sequence, for example, the presence of a read corresponding to each reference sequence in which the marker sequence has altered from the original, indicates that each candidate molecule has caused a response in a cell.

In the series of embodiments described above, in a case where each vector encodes a common candidate molecule, the function of the candidate molecule can be predicted based on the type of expressed reporter system. That is, the response in the cell associated with the expressed reporter system (the response in the cell activating a regulatory sequence operably linked to the reporter system) can be assumed to be caused by the candidate molecule. On the other hand, in a case where each vector encodes a different candidate molecule, while encoding a common regulatory sequence, it allows screening for functional molecules that result in a specific response in a cell.

In a case where a vector molecule includes a unique molecular identifier (UMI), each vector molecule can be individually identified. The sequence of the UMIs can be determined by sequencing. By associating the UMI with the candidate molecule or the reporter, it is possible to reduce bias of the expression level of the reporter associated with sequence amplification caused in the analysis process.

According to the above-described method of the present invention, the response in the cell caused by the candidate molecule can be easily detected by expression of the reporter, and the candidate molecule and the response in the cell caused thereby can be easily associated with each other. Therefore, according to the method of the present invention, the efficiency of functional analysis of functional molecules such as RNA and peptides is improved. Furthermore, in the method of the present invention, two or more kinds of vectors or sets of vectors can be introduced into one cell population, and the behavior of each vector or set of vectors can be analyzed. In this case, in one cell population (for example, in one well), two or more candidate molecules can be examined at a time, or the function of one candidate molecule can be examined from different aspects at a time, so that the throughput of comprehensive analysis of functional molecules can be improved.

The method for searching a functional molecule causing a response in a cell according to the present invention can be applied to drug discovery, diagnosis, and the like. For example, in a case where the present method is applied to drug discovery screening, the vector or set of vectors of the present invention is constructed such that the candidate molecule encoded by the vector or set of vectors is a candidate molecule of a drug and encodes a regulatory sequence that is activated by a response in a cell due to or resulting from the drug effect of interest and a reporter system operably linked thereto. Furthermore, for example, in a case where this method is applied to the search for target genes of drugs, the candidate molecule encoded by the vector or set of vectors of the present invention is a molecule that specifically suppresses a candidate target gene for drug discovery, and the regulatory sequence that is activated by a response in a cell encoded by the vector or set of vectors, and the reporter system operably linked thereto are a system for detecting a signal pathway that is correlated with drug efficacy. As an example, in a case of searching for a therapeutic target of cancer, various shRNAs that inhibit various genes are used as candidate molecules, and a reporter bound to a regulatory sequence (p53 binding sequence (p53RE), antioxidant responsive element (ARE), or the like) that is activated during a stress response of cancer cells, which is considered to be correlated with the efficacy of cancer, is used as a reporter system.

### (Diagnostic Method and Reagent Kit)

In one embodiment, the present invention provides a method of diagnosing a disease, the method including administering a vector or set of vectors of the present invention to a patient or a cell derived from a specimen collected from a patient, and measuring expression of the reporter system from the administered vector or set of vectors. Preferably, the candidate molecule encoded by the vector or the set of vectors is an shRNA that specifically suppresses a causative gene (for example, a mutant gene, a genetic polymorphism, or the like) of a disease to be diagnosed. As a regulatory sequence that is activated by a response in a cell encoded by the vector or the set of vectors, a sequence that responds to an alteration caused by suppression of a causative gene of the disease is used. Specific examples of regulatory sequences include a p53 binding sequence (p53RE), an antioxidant responsive element (ARE), a nuclear factor kappa B (NF-kB) binding sequence, an Activating Transcription Factor 6 responsive element (ATF6 ERSE), a metal regulatory element (MRE), a heat shock element (HSE), a hypoxia responsive element (HRE), a xenobiotic responsive element (XRE), a nuclear factor of activated T-CELLS (NFAT) binding sequence, a TCF/LEF type DNA binding protein binding sequence, sesRNA, or toehold RNA. Expression of the reporter system from the vector or set of vectors indicates activation or aberrant expression of a causative gene of the disease of interest in the patient.

In one embodiment, the present invention provides a companion diagnostic method, the method including administering a vector or set of vectors of the present invention to a patient or a cell derived from a specimen collected from a patient, and measuring expression of the reporter system from the administered vector or set of vectors. In one embodiment, the candidate molecule encoded by the vector or set of vectors is shRNA that complementarily binds to a target gene of a medicament whose effect in the patient is to be examined and suppresses expression thereof. In another embodiment, the candidate molecule encoded by the vector or set of vectors is shRNA that complementarily binds to a mutant (such as a gene polymorphism) of the target gene and suppresses its expression. In another embodiment, the candidate molecule encoded by the vector or set of vectors is an shRNA that complementarily binds to and suppresses the expression of a gene associated with the metabolism of a medicament whose effect in the patient is to be examined. As the regulatory sequence that is activated by a response in a cell encoded by the vector or the set of vectors, a sequence that responds to a biological signal whose activation can be expected to reflect a therapeutic effect is selected. Specific examples of regulatory sequences include a p53 binding sequence (p53RE), an antioxidant responsive element (ARE), a nuclear factor kappa B (NF-kB) binding sequence, an Activating Transcription Factor 6 responsive element (ATF6 ERSE), a metal regulatory element (MRE), a heat shock element (HSE), a hypoxia responsive element (HRE), a xenobiotic responsive element (XRE), a nuclear factor of activated T-CELLS (NFAT) binding sequence, a TCF/LEF type DNA binding protein binding sequence, sesRNA, or toehold RNA. In actual diagnosis, for example, in companion diagnosis for treatment of cancer, in order to clarify a therapeutic target that produces a suppressive effect on a target cancer cell, it is conceivable to use a sequence that responds to intracellular stress such as a p53 responsive sequence or ATF6 ERSE. Expression of the reporter system from the vector or set of vectors is an index for predicting a therapeutic effect when a gene to be a target of a functional molecule is inhibited or activated in the tissue of the patient. For example, in the example of companion diagnosis for cancer treatment, a target gene for applying stress to cancer cells in the patient tissue can be identified. This measurement result can be a stratification marker when a molecular targeting drug of a target gene is prescribed to a cancer patient.

The present invention also provides a reagent kit including the vector or set of vectors of the present invention, that is, a vector or set of vectors including a polynucleotide encoding an expression system for a candidate molecule, a polynucleotide encoding a regulatory sequence activated by a predetermined response in a cell, and a polynucleotide encoding a reporter system operably linked to the regulatory sequence. The reagent kit may include the vector of the present invention, but may further include a reagent for detecting expression of the reporter system from the vector or the set of vectors, a reagent for introducing the vector or the set of vectors into a cell, a pharmaceutically acceptable carrier for administering the vector or the set of vectors to a patient, and the like, as necessary.

In one embodiment, the reagent kit is a diagnostic agent for a disease. In one embodiment, the candidate molecule encoded by the vector or set of vectors used in the diagnostic agent is shRNA or siRNA that complementarily binds to a causative gene (for example, a mutated gene, a genetic polymorphism, or the like) of the disease to be diagnosed and suppresses its expression. In an embodiment, examples of regulatory sequences encoded by the vector or set of vectors include a p53 binding sequence (p53RE), an antioxidant responsive element (ARE), a nuclear factor kappa B (NF-kB) binding sequence, an Activating Transcription Factor 6 responsive element (ATF6 ERSE), a metal regulatory element (MRE), a heat shock element (HSE), a hypoxia responsive element (HRE), a xenobiotic responsive element (XRE), a nuclear factor of activated T-CELLS (NFAT) binding sequence, a TCF/LEF type DNA binding protein binding sequence, sesRNA, or toehold RNA.

In one embodiment, the reagent kit is a companion diagnostic. In one embodiment, the candidate molecule encoded by the vector or set of vectors used in the companion diagnostic agent is shRNA or siRNA that complementarily binds to a target gene of a drug whose effect in a patient is to be examined, or a mutant (such as a genetic polymorphism) of the target gene, and suppresses its expression. In another embodiment, the candidate molecule encoded by the vector or set of vectors used in the companion diagnostic agent is shRNA or siRNA that complementarily binds to a gene associated with the metabolism of a drug whose effect in a patient is to be examined, and suppresses its expression. In an embodiment, examples of regulatory sequences encoded by the vector or set of vectors include a p53 binding sequence (p53RE), an antioxidant responsive element (ARE), a nuclear factor kappa B (NF-kB) binding sequence, an Activating Transcription Factor 6 responsive element (ATF6 ERSE), a metal regulatory element (MRE), a heat shock element (HSE), a hypoxia responsive element (HRE), a xenobiotic responsive element (XRE), a nuclear factor of activated T-CELLS (NFAT) binding sequence, a TCF/LEF type DNA binding protein binding sequence, sesRNA, or toehold RNA.

### (Method for Analyzing Activity of Regulatory Sequence)

The present invention also provides a method for analyzing the activity of a regulatory sequence (a transcriptional regulatory sequence or a translational regulatory sequence). In this analysis method, a vector including a polynucleotide encoding a regulatory sequence to be analyzed, a polynucleotide encoding a reporter system operably linked to the regulatory sequence, and a label sequence for identifying the regulatory sequence are used. In this analysis method, a predetermined response in a cell is elicited in a cell into which the vector has been introduced, and then the expression of the reporter system encoded by the vector is quantified. The expression of the reporter system and the activity of the regulatory sequence to the predetermined response in the cell are assessed based on the label sequence.

The regulatory sequence encoded by the vector is not particularly limited as long as it is a transcriptional regulatory sequence or a translational regulatory sequence whose activity for a predetermined response in a cell is desired to be analyzed. The type of the predetermined response in the cell is not particularly limited, and examples thereof include a DNA damage response, an oxidative stress response, and specific protein or mRNA expression. Preferably, the regulatory sequence is a transcriptional regulatory sequence. Preferably, the transcriptional regulatory sequence is a promoter or promoter variant.

Examples of the label sequence included in the vector for identifying the regulatory sequence include an identifier sequence that identifies each type of regulatory sequence, and a molecular identifier sequence that uniquely identifies a regulatory sequence (UMI). For example, different identifier sequences are assigned for each type, structure, or sequence of regulatory sequences. Alternatively, the label sequence may be present in the region of the regulatory sequence. For example, a regulatory sequence variant having a mutation in the sequence may itself be a label sequence.

Examples of reporter systems encoded by the vector include luminescent or fluorescent protein reporter systems, enzymatic reporter systems, and the like. Examples of enzymes expressed by the enzymatic reporter system include DNA nucleases, DNA recombinases, hydrolases (for example, β-glucuronidase), transposases, integrases, and the like. Specific examples of the enzymatic reporter system are as described above.

In one preferred embodiment, the reporter system includes a protein having an action of changing a target nucleotide sequence and a target sequence thereof, and expression of the reporter system changes the target sequence. By measuring the amount of alteration in the target sequence, the expression of the reporter system can be quantified. Preferred examples of such reporter systems include the aforementioned CRE-LoxP-like system, piggyBac transposon system, phiC31/attP system, Base Editor or Prime Editor and recognition sequences thereof, and the like. The CRE-LoxP-like system consists of a CRE recombinase and two LoxP-like sequences with a marker sequence located between them. The two LoxP-like sequences may be arranged in series in the same direction, or may be arranged in series in opposite directions. When CRE is expressed, if the LoxP-like sequences are arranged in the same orientation, the marker sequence in between is excised from the vector, while if the LoxP sequences are arranged in opposite directions, the marker sequence on the vector is inverted. The piggyBac transposon system is composed of a piggyBac transposase, two ITRs, and a marker sequence in therebetween. When the transposase is expressed, the marker sequence between the ITRs is transferred (disappeared from between the ITRs). Therefore, by examining the alteration or deletion of the target sequence (the marker sequence or the enzyme recognition sequence) in the vector, the expression of the reporter system can be quantified.

Specifically, this analysis method includes preparing a cell into which the vector is introduced; in the cell, eliciting a predetermined response in a cell; after eliciting the response, analyzing the target sequence and the region including the label sequence in the vector and quantifying the alteration in the target sequence;
associating the target sequence with the transcriptional regulatory sequence or the translational regulatory sequence based on the label sequence; and
evaluating the activity of the transcriptional regulatory sequence or the translational regulatory sequence associated with the target sequence against the predetermined response in the cell based on the amount of alteration in the target sequence.

Examples of the type of vector that can be used in the present analysis method, the type of cell into which the vector is to be introduced (host cell), and the method for introducing the vector into the cell include those that can be used in the above-described method for searching a functional molecule causing a response in a cell.

In the present analysis method, the means for eliciting the predetermined response in the cell is not particularly limited, and examples thereof include application of an agent or stimulus that induces DNA damage or oxidative stress, application of a signaling pathway activating agent such as Wnt pathway, suppression of a gene or protein involved in DNA repair or antioxidant, activation of a gene or protein related to a specific signaling pathway, activation or suppression of a gene related to expression of a target protein or mRNA, and the like. Means for activating or suppressing such a drug or stimulus, or a gene or protein of interest are known in the art.

The means for analyzing the region including the target sequence and the label sequence in the vector may be any method capable of detecting and quantifying a alteration in the nucleotide sequence, and examples thereof include quantitative RT-PCR, quantitative PCR, sequencing, and the like.

The amount of alteration in the target sequence reflects the expression level of the reporter system and thus the activity of the regulatory sequence. On the other hand, since the label sequence present in the same region as the target sequence specifies the regulatory sequence that has caused the alteration in the target sequence, the target sequence and the regulatory sequence can be associated with each other based on the label sequence. Therefore, the activity of the regulatory sequence associated with the target sequence against the predetermined response in the cell can be evaluated based on the amount of alteration in the target sequence. For example, the greater the amount of alteration in the target sequence that occurs following a predetermined response within a cell, the more strongly the regulatory sequence associated with the target sequence is activated against the predetermined response within the cell can be assessed.

This analysis method includes introducing at least one type of vector into a cell and measuring the expression of a marker or reporter system from the vector. The phrase "introducing at least one type of vector into a cell" is as defined above. Preferably, in this analysis method, a pool of a plurality (two or more) of vectors each including a polynucleotide encoding a different regulatory sequence is used, and the activity of each regulatory sequence is comprehensively analyzed. For example, in the method of the present invention, after a pool of the vectors is added to one cell population and each vector is introduced into any cell in the cell population, the alteration in the target sequence in each vector is comprehensively quantified. For example, by collectively sequencing the label sequences derived from different vectors and regions including target sequences, alterations in each target sequence can be quantified, and regulatory sequences that have caused alterations in each target sequence can be identified based on the label sequences.

### (Method for searching Gene involved in Response in Cell)

The present invention also provides a method for searching a gene involved in a response in a cell. The method includes the follows:
introducing a plurality of vectors or a set of vectors into a cell population, in which the plurality of vectors or the set of vectors each encodes the follows:
   RNA that interferes with a gene of interest,
   a regulatory sequence that is activated by a predetermined response in a cell, and
   a reporter system operably linked to the regulatory sequence,
where the RNA encoded by each of the plurality of vectors or the set of vectors being different from each other;
measuring expression of the reporter system encoded by each of the plurality of vectors or the set of vectors in the cell population; and
determining whether the gene of interest is a gene involved in the predetermined response in the cell based on the expression of the reporter system contained in each of the plurality of vectors or the set of vectors.

Therefore, the vector or set of vectors used in the present method encodes RNA that interferes with the gene of interest, a regulatory sequence that is activated by a predetermined response in a cell whose relationship with the gene of interest is desired to be examined, and a reporter system operably linked to the regulatory sequence. In other words, the vector or set of vectors includes a polynucleotide encoding an expression system for the RNA, a polynucleotide encoding the regulatory sequence, and a polynucleotide encoding the reporter system. These polynucleotides may be arranged on the same vector or on different vectors. The arrangement of the polynucleotide encoding the expression system of the RNA, the polynucleotide encoding the regulatory sequence, or the polynucleotide encoding the reporter system on each vector is not particularly limited, as long as the RNA is expressed and the expression of the reporter system is promoted by activation of the regulatory sequence.

Examples of the RNA that interferes with the gene of interest and is encoded by the vector or the set of vectors include shRNA, gRNA, miRNA, saRNA, lncRNA, and eRNA. Among them, shRNA and gRNA are preferable. The expression system of the RNA may include a sequence encoding the RNA and a transcriptional regulatory sequence such as a promoter operably linked thereto. The gene of interest is not particularly limited as long as it is a gene whose relationship with the predetermined response in the cell is desired to be examined. The type of response in the given cell is not particularly limited, and examples thereof include a DNA damage response, an oxidative stress response, and specific protein or mRNA expression.

The regulatory sequence encoded by the vector or the set of vectors is not particularly limited as long as it is a transcriptional regulatory sequence or a translational regulatory sequence that is activated by a predetermined response in a cell whose relationship with the gene of interest is desired to be examined. In addition, the reporter system encoded by the vector or the set of vectors is not particularly limited as long as it can be expressed under the regulation of the regulatory sequence. Examples of the regulatory sequence and reporter system include those exemplified above as the regulatory sequence and reporter system that can be encoded by the vector used in the method for searching a functional molecule that causes a response in a cell. The activity of the regulatory sequence and the level of expression of the reporter system regulated thereby reflect the magnitude of the predetermined response in the cell due to the introduction of the RNA.

The plurality of vectors or sets of vectors used in the present method are preferably three or more kinds, more preferably four or more kinds of vectors or sets of vectors. The RNA encoded by each of the plurality of vectors or sets of vectors interferes with a common gene of interest, but is different from each other. For example, the RNA targets a common gene of interest, and is shRNA or gRNA composed of different sequences.

The reporter system encoded by the plurality of vectors or sets of vectors is constructed so that it can be identified from which of the RNAs encoded by the plurality of vectors or sets of vectors the reporter system is expressed. Preferably, the reporter system encoded by the plurality of vectors or sets of vectors is labeled so that its expression is associated with the RNA encoded by the same vector or set of vectors.

In the method, the expression of the reporter system encoded by each of the plurality of vectors or sets of vectors is measured. Then, it is determined whether the gene of interest is a gene involved in the predetermined response in the cell based on the measured expression of each reporter system.

In one embodiment, this method comprehensively analyzes the magnitude of the predetermined response in the cell due to the introduction of the RNA encoded by each of the plurality of vectors or sets of vectors. For example, in the method of the present invention, a pool of the plurality of vectors or sets of vectors is added to a cell population, and each vector or set of vectors is introduced into any cell in the cell population, and then expression of a reporter from each vector or set of vectors is measured. Changes in a predetermined response in a cell due to each of the RNAs can be comprehensively analyzed based on the association of the reporter system with the RNA.

Regardless of the type of the introduced RNA, in a case where a change in a predetermined response (for example, promotion or suppression of response) in the cell has occurred, it can be determined that the gene of interest by the RNA is (either in a pro-active or suppressive manner) involved in the predetermined response in the cell. On the other hand, in a case where only a particular type of RNA has resulted in an altered predetermined response in the cell, the gene of interest that the RNA targets is likely not involved in the predetermined response in the cell.

In one embodiment, whether the gene of interest is a gene involved in the predetermined response in the cell is determined based on the statistical value of expression for the reporter system included in each of the plurality of vectors or set of vectors (in other words, the change in the predetermined response in the cell due to the introduction of RNA linked to the reporter system). For example, the mean value or median value of expression of all reporter systems can be calculated, and the gene of interest can be ranked by the value. Alternatively, if the expression of a majority of reporter systems is changed in the same fashion (for example, either in a pro-active or suppressive manner), the gene of interest can be determined to be involved in the predetermined response within the cell.

Examples of the types of vectors that can be used in this method, the types of cells (host cells) into which the vectors are introduced, the methods for introducing the vectors into the cells, and the means for measuring the expression of the reporter system include the same as those that can be used in the method for searching a functional molecule causing a response in a cell described above.

### [Examples]

Hereinafter, the present invention will be described in more detail by way of examples. However, the present invention is not limited at all by these examples and the like.

Primers and templates for PCR used in the following examples are shown in Tables 1 to 7 and Tables A1 to J.

**[Table 1]**

| Table 1 Primers for Preparing Plasmid 1 | | | |
|---|---|---|---|
| SEQ ID No. | Primer | Sequence (5'-3') | Template |
| 1 | Forward | GTTAAGCCAGCCCCGACACAATAAAATATCTTTATTTTCATTAC ATCTGTGTGTTGG | p53RE-CRE |
| 2 | Reverse | ctttgtacaagaaagctgggtCTAATCGCCATCTTCCAGC | |
| 3 | Forward | GCTGGAAGATGGCGATTAGacccagctttcttgtacaaag | SV40 late pA |
| 4 | Reverse | CACTGACGGGCACCGGAGCCgcgttaattaaTACCACATTTG | |
| 5 | Forward | GTAttaattaacgcGGCTCCGGTGCCCGTCAGTG | EF1A promoter-Lox2272-LoxP-mKate2 (Reverse compliment)-Lox2272-LoxP-SV40 late pA |
| 6 | Reverse | gaatcatgggaaataggccctcTACCACATTTGTAGAGGTTTTAC | |
| 7 | Forward | GTAAAACCTCTACAAATGTGGTAgagggcctatttcccatgattc | U6-shScramble-terminater-PGK promoter-EGFP-rGB pA |
| 8 | Reverse | CTATGCGGCATCAGAGCAGgatctccataagagaagagg | |
| 9 | Forward | cctcttctcttatggagatcCTGCTCTGATGCCGCATAG | OriC |
| 10 | Reverse | GAAAATAAAGATATTTTATTGTGTCGGGGCTGGCTTAAC | |

**[Table 2]**

| Table 2 Primers for Preparing Plasmid 2 | | | |
|---|---|---|---|
| SEQ ID No. | Primer | Sequence (5'-3') | Template |
| 1 | Forward | GTTAAGCCAGCCCCGACACAATAAAATATCTTTATTTTCAT TACATCTGTGTGTTGG | p53RE-CRE |
| 2 | Reverse | ctttgtacaagaaagctgggtCTAATCGCCATCTTCCAGC | |
| 3 | Forward | GCTGGAAGATGGCGATTAGacccagctttcttgtacaaag | SV40 late pA |
| 4 | Reverse | CACTGACGGGCACCGGAGCCgcgttaattaaTACCACATTTG | |
| 5 | Forward | GTAttaattaacgcGGCTCCGGTGCCCGTCAGTG | EF1A promoter-Lox2272-LoxP-mKate2 (Reverse compliment)-Lox2272-LoxP-SV40 late pA |
| 6 | Reverse | gaatcatgggaaataggccctcTACCACATTTGTAGAGGTTTTAC | |
| 7 | Forward | GTAAAACCTCTACAAATGTGGTAgagggcctatttcccatgattc | U6-shRAD51_1-terminater |
| 11 | Reverse | aagttgatgatatcgaattcaaaaaGAAGCTATGTTCGCCATTACTC GAGTAATGGCGAACATAGCTTCccggtgtttcgtcctttcc | |
| 7 | Forward | GTAAAACCTCTACAAATGTGGTAgagggcctatttcccatgattc | U6-shRA051_2-terminater |
| 12 | Reverse | aagttgatgatatcgaattcaaaaaGCCAACGATGTGAAGAAATTCT CGAGAATTTCTTCACATCGTTGGCccggtgtttcgtcctttcc | |
| 7 | Forward | GTAAAACCTCTACAAATGTGGTAgagggcctatttcccatgattc | U6-shRA051_3-terminater |
| 13 | Reverse | aagttgatgatatcgaattcaaaaaAAGCTATGTTCGCCATTAACTCG AGTTAATGGCGAACATAGCTTccggtgtttcgtcctttcc | |
| 7 | Forward | GTAAAACCTCTACAAATGTGGTAgagggcctatttcccatgattc | U6-shRA051_4-terminater |
| 14 | Reverse | aagttgatgatatcgaattcaaaaaGCAGTGATGTCCTGGATAACTC GAGTTATCCAGGACATCACTGCccggtgtttcgtcctttcc | |
| 15 | Forward | tttttgaattcgatatcatcaactttgtatag | PGK promoter-EGFP-rGB pA |
| 8 | Reverse | CTATGCGGCATCAGAGCAGgatctccataagagaagagg | |
| 9 | Forward | cctcttctcttatggagatcCTGCTCTGATGCCGCATAG | OriC |
| 10 | Reverse | GAAAATAAAGATATTTTATTGTGTCGGGGCTGGCTTAAC | |

**[Table 3]**

| Table 3 Primers for Preparing Plasmid 3 | | | |
|---|---|---|---|
| SEQ ID No | Primer | Sequence (5'-3') | Template |
| 1 | Forward | GTTAAGCCAGCCCCGACACAATAAAATATCTTTATTTTCATTACATCTGTGTGTTGG | part of vector 1 |
| 16 | Reverse | CAGTTAGGCCAGAGAAATGTTCTGGC | |
| 17 | Forward | GGCTGTCCCCAGTGCAAGTG | ARE |
| 18 | Reverse | GGTGGCTTTACCAACAGTACCG | |
| 19 | Forward | GCTCGACTTCCAGCTTGGC | part of vector 1 |
| 4 | Reverse | CACTGACGGGCACCGGAGCCgcgttaattaaTACCACATTTG | |
| 5 | Forward | GTAttaattaacgcGGCTCCGGTGCCCGTCAGTG | EF1A promoter-Lox2272-LoxP-mKate2 (Reverse compliment)-Lox2272-LoxP-SV40 late pA |
| 6 | Reverse | gaatcatgggaaataggccctcTACCACATTTGTAGAGGTTTTAC | |
| 7 | Forward | GTAAAACCTCTACAAATGTGGTAgagggcctatttcccatgattc | U6-shScramble-terminater-PGK promoter-EGFP-rGB pA |
| 8 | Reverse | CTATGCGGCATCAGAGCAGgatctccataagagaagagg | |
| 9 | Forward | cctcttctcttatggagatcCTGCTCTGATGCCGCATAG | OriC |
| 10 | Reverse | GAAAATAAAGATATTTTATTGTGTCGGGGCTGGCTTAAC | |

**[Table 4]**

| Table 4 Primers for Calculating Inversion Rate of mKate2 sequences | | | |
|---|---|---|---|
| Target | Primer | Sequence (5'-3') | SEQ ID No. |
| Original | Forward | ATTTCAGGTGTCGTGACAAGTTTGTAC | 20 |
| | Reverse | TCCCTAGCAAACTGGGGCAC | 21 |
| Flip | Forward | ATTTCAGGTGTCGTGACAAGTTTGTAC | 20 |
| | Reverse | GAAGTGGTGGTTGTTCACGG | 22 |
| Backbone (OriC sequence-specific primers) | Forward | TCAGGAAGCTTGGATCAACCGG | 23 |
| | Reverse | TGATCGGTGATCCTGGACCG | 24 |

**[Table 5]**

| Table 5 Primers for Preparing Amplicon (Common to Plasmids 1 and 2) | | |
|---|---|---|
| Primer | Sequence (5'-3') | SEQ ID No. |
| Forward | GGCCAGCTTGGCACTTGATG | 25 |
| Reverse | GGACGTGAAGAATGTGCGAG | 26 |

**[Table 6]**

| Table 6 Primers for Preparing Amplicons for each shRNA | | | |
|---|---|---|---|
| Amplicon | Primer | Sequence (5'-3') | SEQ ID No. |
| Fragments containing shScramble | Forward | GGCCAGCTTGGCACTTGATG | 25 |
| | Reverse | GAGGGCGACTTAACCTTAGG | 27 |
| Fragments containing shRAD51_2 | Forward | GGCCAGCTTGGCACTTGATG | 25 |
| | Reverse | AATTTCTTCACATCGTTGGC | 28 |
| Fragments containing shRAD51_4 | Forward | GGCCAGCTTGGCACTTGATG | 25 |
| | Reverse | TTATCCAGGACATCACTGC | 29 |

**[Table 7]**

| Table 7 Primers for Calculating Inversion Rate of mKate2 sequences | | | |
|---|---|---|---|
| Target | Primer | Sequence (5'-3') | SEQ ID No. |
| Original | Forward | ATTTCAGGTGTCGTGACAAGTTTGTAC | 20 |
| | Reverse | TCCCTAGCAAACTGGGGCAC | 21 |
| Flip | Forward | ATTTCAGGTGTCGTGACAAGTTTGTAC | 20 |
| | Reverse | GAAGTGGTGGTTGTTCACGG | 22 |

**[Table A1]**

| Table A1 Primers for Preparing Linear DNA 1 | | |
|---|---|---|
| SEQ ID No | Primer | Sequence (5'-3') |
| 30 | Forward | GTTAAGCCAGCCCCGACACTGTTCACGTAGAGGAGGTTATCC |
| 31 | Reverse | CTATGCGGCATCAGAGCAGGTACCGTTATATGCCGGCGG |

**[Table A2]**

| Table A2 Primers for Preparing Linear DNA 2 | | |
|---|---|---|
| SEQ ID No | Primer | Sequence (5'-3') |
| 30 | Forward | GTTAAGCCAGCCCCGACACTGTTCACGTAGAGGAGGTTATCC |
| 32 | Reverse | CGGTGTTTCGTCCTTTCCACAAGATATATAAAGCC |
| 33 | Forward | tagtacagtagtaatagtatatgtgatggaTC |
| 31 | Reverse | CTATGCGGCATCAGAGCAGGTACCGTTATATGCCGGCGG |

**[Table B]**

| Table B shRNA for Linear DNA 2A/Linear DNA 3B (underlined regions indicate homologous regions of target sequence) | | | |
|---|---|---|---|
| shRNA ID | Target gene | Sequence (5'-3') | SEQ ID No |
| USP7_1 | USP7 | TCTTGGCTTTATATATCTTGTGGAAAGGACGAAACACCGGCCAGCTAAGTATCAAAG GAAACTCGAGTTTCCTTTGATACTTAGCTGGTTTTTTAGTACAGTAGTAATAGTATATG TGATGGATCT | 34 |
| USP7_2 | USP7 | TCTTGGCTTTATATATCTTGTGGAAAGGACGAAACACCGGCCAGCTAAGTATCAAAG GAAACTCGAGTTTCCTTTGATACTTAGCTGGTTTTTTAGTACAGTAGTAATAGTATATG TGATGGATCT TCTTGGCTTTATATATCTTGTGGAAAGGACGAAACACCGGCCTGGATTTGTGGTTAC GTTACTCGAGTAACGTAACCACAAATCCAGGTTTTTTAGTACAGTAGTAATAGTATAT GTGATGGATCT TCTTGGCTTTATATATCTTGTGGAAAGGACGAAACACCGGGTGTCCTATATCCAGTG TAAACTCGAGTTTACACTGGATATAGGACACTTTTTTAGTACAGTAGTAATAGTATATG TGATGGATCT | 35 |
| USP7_3 | USP7 | TCTTGGCTTTATATATCTTGTGGAAAGGACGAAACACCGGGTGTCCTATATCCAGTG TAAACTCGAGTTTACACTGGATATAGGACACTTTTTTAGTACAGTAGTAATAGTATATG TGATGGATCT | 36 |
| USP7_4 | USP7 | TCTTGGCTTTATATATCTTGTGGAAAGGACGAAACACCGGCGTGGTGTCAAGGTGTA CTAACTCGAGTTAGTACACCTTGACACCACGTTTTTTAGTACAGTAGTAATAGTATAT GTGATGGATCT | 37 |
| DHX29_1 | DHX29 | TCTTGGCTTTATATATCTTGTGGAAAGGACGAAACACCGGCGTGTACCTTTGGAGGA ATTACTCGAGTAATTCCTCCAAAGGTACACGTTTTTTAGTACAGTAGTAATAGTATATG TGATGGATCT | 38 |
| DHX29_2 | DHX29 | TCTTGGCTTTATATATCTTGTGGAAAGGACGAAACACCGGCCTAAGTATCAGAAACT TCTACTCGAGTAGAAGTTTCTGATACTTAGGTTTTTTAGTACAGTAGTAATAGTATATG TGATGGATCT | 39 |
| DHX29_3 | DHX29 | TCTTGGCTTTATATATCTTGTGGAAAGGACGAAACACCGGCCTGGTAGTATGCCCTA CAATCTCGAGATTGTAGGGCATACTACCAGGTTTTTTAGTACAGTAGTAATAGTATATG TGATGGATCT | 40 |
| DHX29_4 | DHX29 | TCTTGGCTTTATATATCTTGTGGAAAGGACGAAACACCGGCCTCAAATACAAGCCAC TATTCTCGAGAATAGTGGCTTGTATTTGAGGTTTTTTAGTACAGTAGTAATAGTATATG TGATGGATCT | 41 |
| SETDB1_1 | SETDB1 | TCTTGGCTTTATATATCTTGTGGAAAGGACGAAACACCGGCGTGACTTCATAGAGGA GTATCTCGAGATACTCCTCTATGAAGTCACGTTTTTTAGTACAGTAGTAATAGTATATG TGATGGATCT | 42 |
| SETDB1_2 | SETDB1 | TCTTGGCTTTATATATCTTGTGGAAAGGACGAAACACCGGGCTCAGATGATAACTTC TGTACTCGAGTACAGAAGTTATCATCTGAGCTTTTTTAGTACAGTAGTAATAGTATATG TGATGGATCT | 43 |
| SETDB1_3 | SETDB1 | TCTTGGCTTTATATATCTTGTGGAAAGGACGAAACACCGGAGTTAGAGACATGGGTA ATACCTCGAGGTATTACCCATGTCTCTAACTTTTTTTAGTACAGTAGTAATAGTATATG TGATGGATCT | 44 |

**[Table C1]**

| Table C1 Primers for Preparing Linear DNA 3A | | |
|---|---|---|
| SEQ ID No | Primer | Sequence (5'-3') |
| 45 | Forward | TGTTCACGTAGAGGAGGTTATCC |
| 46 | Reverse | GTACCGTTATATGCCGGCGG |

**[Table C2]**

| Table C2 Primers for Preparing Linear DNA 3B | | |
|---|---|---|
| SEQ ID No | Primer | Sequence (5'-3') |
| 45 | Forward | TGTTCACGTAGAGGAGGTTATCC |
| 47 | Reverse | CCGGTGTTTCGTCCTTTCCACAAG |
| 48 | Forward | TAGTACAGTAGTAATAGTATATGTGATGGATC |
| 46 | Reverse | GTACCGTTATATGCCGGCGG |

**[Table D]**

| Table D Primers for Analysis of Wnt Pathway-related Genes | | | |
|---|---|---|---|
| SEQ ID No | Primer | Sequence (5'-3') | Target gene |
| 49 | Forward | GTCTCCTCTGACTTCAACAGCG | GAPDH |
| 50 | Reverse | ACCACCCTGTTGCTGTAGCCAA | |
| 51 | Forward | CTGGCTTTGGTGAACTGTTG | AXIN2 |
| 52 | Reverse | AGTTGCTCACAGCCAAGACA | |
| 53 | Forward | GAAACAGTGCTCGGGTTTTC | SP5 |
| 54 | Reverse | TAGCTCTGCATGGAGCTGAA | |

**[Table E]**

| Table E Primers for Analysis of Lox Intersequences | | | |
|---|---|---|---|
| SEQ ID No | Primer | Sequence (5'-3') | Target gene |
| 55 | Forward | TGATCAACCGTGGTTACAAGCAGG | Lox |
| 56 | Reverse | CTGGACTGTACCTCGTTGTACGG | |
| 57 | Forward | GGCCTATTTCCCATGATTCCTTC | backbone |
| 58 | Reverse | CCAAGAAATTATTACTTTCTACGTCACG | |

**[Table F]**

| Table F Primers for Amplifying Lox Intersequences | | |
|---|---|---|
| SEQ ID No | Primer | Sequence (5'-3') |
| 25 | Forward | GGCCAGCTTGGCACTTGATG |
| 59 | Reverse | AAAGCCATACGGGAAGCAATAG |

**[Table G]**

| Table G Primers for Amplifying Lox Intersequences-shRNA coding sequences | | |
|---|---|---|
| SEQ ID No | Primer | Sequence (5'-3') |
| 60 | Forward | AATAGGGCCGGTGGAAAGGACGAAACACCG |
| 61 | Reverse | TGACTCGTCGCCAGTTCCTACATTCGCTCTC |
| 62 | Forward | CAACGGCTACGTGGAAAGGACGAAACACCG |
| 63 | Reverse | CTCCATGTTGCCAGTTCCTACATTCGCTCTC |
| 64 | Forward | CAGCGAATGCGTGGAAAGGACGAAACACCG |
| 65 | Reverse | CTACAAGGATCCAGTTCCTACATTCGCTCTC |

**[Table H1]**

| Table H1 Primers for Preparing Linear DNA 4 | | |
|---|---|---|
| SEQ ID No | Primer | Sequence (5'-3') |
| 45 | Forward | TGTTCACGTAGAGGAGGTTATCC |
| 66 | Reverse | AGATCCATCACATATACTATTACTACTGTAC |
| 45 | Forward | TGTTCACGTAGAGGAGGTTATCC |
| 47 | Reverse | CCGGTGTTTCGTCCTTTCCACAAG |
| 67 | Forward | TGCGACTGACGGTTGCAACG |
| 46 | Reverse | GTACCGTTATATGCCGGCGG |

**[Table H2]**

| Table H2 Primers for Amplifying Linear DNA 4 | | |
|---|---|---|
| SEQ ID No | Primer | Sequence (5'-3') |
| 45 | Forward | TGTTCACGTAGAGGAGGTTATCC |
| 46 | Reverse | GTACCGTTATATGCCGGCGG |

**[Table I]**

| Table I Primers for Preparing Plasmid 7 | | | |
|---|---|---|---|
| SEQ ID No. | Primer | Sequence (5'-3') | Template |
| 1 | Forward | GTTAAGCCAGCCCCGACACAATAAAATATCTTTATTTTCATTA CATCTGTGTGTTGG | p53RE-CRE |
| 68 | Reverse | AGTGTCTAAGCTTGGCCGC | |
| 69 | Forward | GGATATCAAGATCTGGCCTCG | minP variant |
| 70 | Reverse | GGGCCCATGGTGGCTTTAC | |
| 71 | Forward | CTTGGCAATCCGGTACTGTTG | CRE |
| 2 | Reverse | ctttgtacaagaaagctgggtCTAATCGCCATCTTCCAGC | |
| 3 | Forward | GCTGGAAGATGGCGATTAGacccagctttcttgtacaaag | SV40 late pA |
| 4 | Reverse | CACTGACGGGCACCGGAGCCgcgttaattaaTACCACATTTG | |
| 5 | Forward | GTAttaattaacgcGGCTCCGGTGCCCGTCAGTG | EF1A promoter-Lox2272-LoxP-mKate2 (Reverse compliment)-Lox2272-LoxP-SV40 late pA |
| 6 | Reverse | gaatcatgggaaataggccctcTACCACATTTGTAGAGGTTTTAC | |
| 7 | Forward | GTAAAACCTCTACAAATGTGGTAgagggcctatttcccatgattc | U6-shScramble-terminater-PGK |
| 8 | Reverse | CTATGCGGCATCAGAGCAGgatctccataagagaagagg | promoter-EGFP-rGB pA |
| 9 | Forward | cctcttctcttatggagatcCTGCTCTGATGCCGCATAG | OriC |
| 10 | Reverse | GAAAATAAAGATATTTTATTGTGTCGGGGCTGGCTTAAC | |

**[Table J]**

| Table J Primers for Amplifying minP Variant Sequence-mKate2 Sequence | | |
|---|---|---|
| SEQ ID No | Primer | Sequence (5'-3') |
| 69 | Forward | GGATATCAAGATCTGGCCTCG |
| 26 | Reverse | GGACGTGAAGAATGTGCGAG |

### Example 1 Preparation of Vector

### 1) Preparation of Plasmid 1

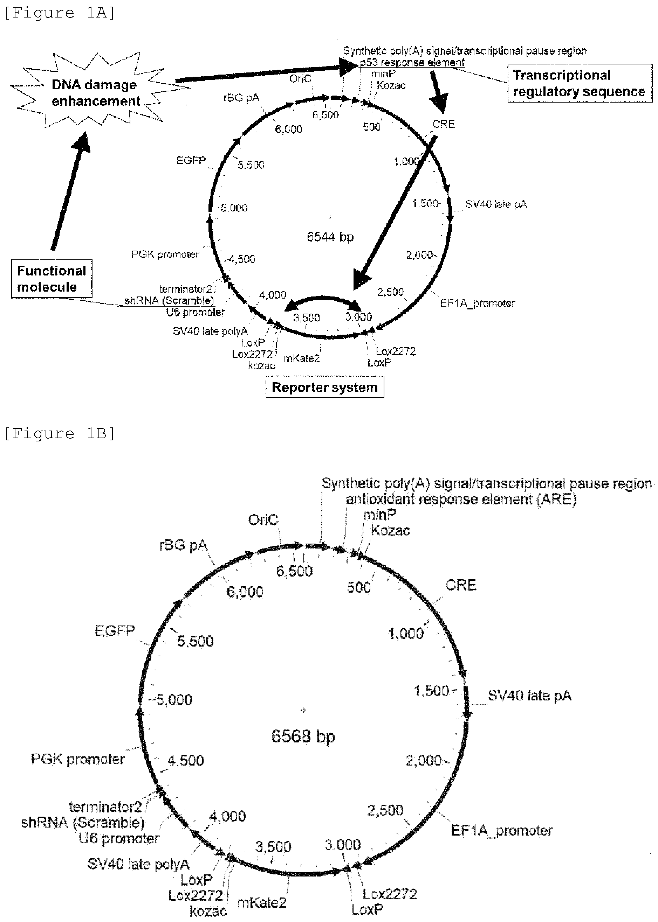

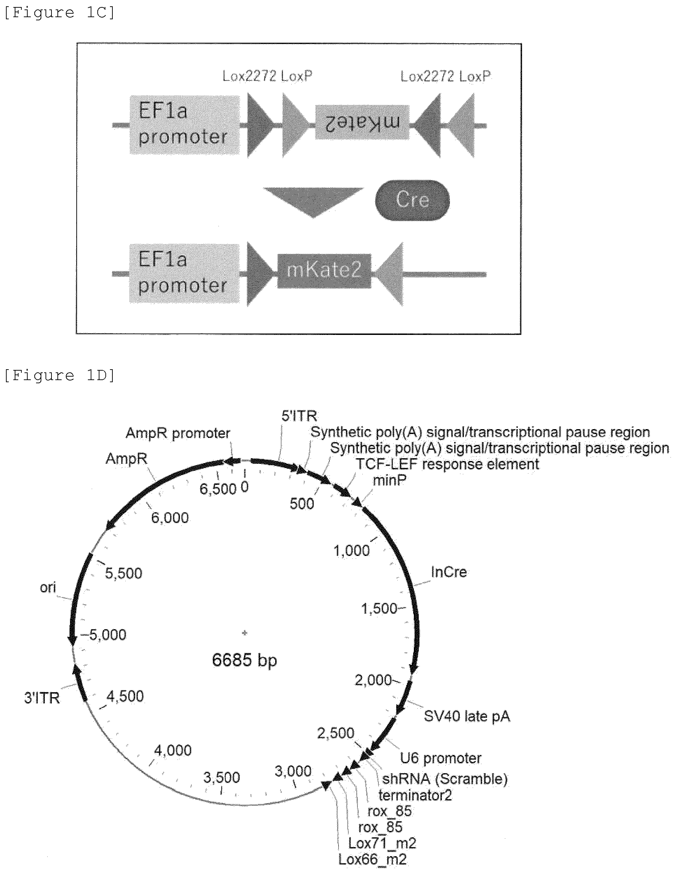

PCR was implemented using a combination of a template and a primer shown in Table 1. Platinum^{™} SuperFi II PCR Master Mix (ThermoFisher) was used for PCR. PCR products were subjected to agarose gel electrophoresis. In a case where a single band was detected by electrophoresis, a PCR reaction solution was directly added to a column of Wizard^{™} SV Gel and PCR Clean-Up System (Promega), and a PCR fragment was purified according to a manufacturer's recommended protocol. In a case where a nonspecific sequence was detected by electrophoresis, a gel including a target fragment was cut out and subjected to Wizard^{™} SV Gel and PCR Clean-Up System (Promega) to purify the PCR fragment. The purified fragments were assembled using Cell-Free Cloning System (OriCiro) according to the manufacturer's protocol, and amplified in vitro. The obtained reaction solution was directly added to a column of Wizard^{™} SV Gel and PCR Clean-Up System (Promega), and a plasmid vector (Plasmid 1) was purified according to a manufacturer's recommended protocol. The obtained plasmid was cleaved with XbaI (New England Biolabs) and the molecular weight was confirmed by agarose gel electrophoresis. This plasmid had a sequence encoding a scrambled shRNA (sh-scramble) not having a target in the region of the functional molecule, had p53RE as a transcriptional regulatory sequence, and had a CRE-loxP-like sequence as a reporter system downstream of the transcriptional regulatory sequence. The CRE-loxP-like sequence had a CRE gene sequence, two Lox2722-LoxP sequences arranged in opposite directions to each other, and a red fluorescent protein mKate2 coding sequence (mKate2 sequence) arranged therebetween (Fig. 1A). In its original state, the mKate2 sequence is positioned in the opposite direction to the transcription direction and is not expressed. When the transcriptional regulatory sequence is activated, the mKate2 sequence is inverted by the action of CRE and becomes transcribable under the action of the upstream EF1 promoter (Fig. 1C). Cells expressing mKate2 were labeled with red fluorescence, indicating that the functional molecule generated a response in a cell activating p53RE. The plasmid had GFP linked to a constitutively expressed promoter, and the transformant having the plasmid was labeled with green fluorescence.

### 2) Preparation of Plasmid 2

A plasmid vector (Plasmid 2) was prepared in the same procedure as in Plasmid 1 except that PCR was implemented using a combination of a template and a primer shown in Table 2, and the molecular weight of the plasmid vector was confirmed. This plasmid had a sequence encoding shRNA targeting RAD51 (shRAD51) in the region of the functional molecule, and as described above, had a CRE-loxP-like sequence including a transcriptional regulatory sequence and p53RE as a reporter system, and an mKate2 sequence (Fig. 1A). The shRAD51 in the plasmid was any of 4 kinds (shRAD51_1 to shRAD51_4) shown in Table 2. Four plasmids with each shRAD51 sequence were pooled and used as Plasmid 2. Similar to Plasmid 1, the mKate2 sequence in Plasmid 2 is inverted and expressed by activation of transcriptional regulatory sequences (Fig. 1C), whereby cells are labeled with red fluorescence. The plasmid had GFP linked to a constitutively expressed promoter, and the transformant having the plasmid was labeled with green fluorescence.

### 3) Preparation of Plasmid 3

A plasmid vector (Plasmid 3) was prepared in the same procedure as in Plasmid 1 except that PCR was implemented using a combination of a template and a primer shown in Table 3, and the molecular weight of the plasmid vector was confirmed. This plasmid had a sh-scramble in the region of the functional molecule, had Antioxidant responsive element (ARE) as a transcriptional regulatory sequence, and had a CRE-loxP-like sequence as a reporter system downstream of the transcriptional regulatory sequence. The CRE-loxP-like sequence had a CRE gene sequence and two Lox2722-LoxP sequences arranged in the opposite direction to each other with the mKate2 coding sequence arranged therebetween (Fig. 1B). Similar to Plasmid 1, the mKate2 sequence in Plasmid 3 is inverted and expressed by activation of transcriptional regulatory sequences (Fig. 1C), whereby cells are labeled with red fluorescence. The plasmid had GFP linked to a constitutively expressed promoter, and the transformant having the plasmid was labeled with green fluorescence.

### 4) Preparation of Plasmid 4

A plasmid vector having the structure of Fig. 1D was prepared by GenScript Japan Co., Ltd. This plasmid had sh-scramble in the functional molecular region, a TCF/LEF DNA-binding protein binding sequence as a transcriptional regulatory sequence, and a CRE-loxP-like sequence as a reporter system downstream of the transcriptional regulatory sequence. The CRE-loxP-like sequence had a CRE gene sequence and a Lox71 sequence and a Lox66 sequence arranged in opposite directions to each other (Lee et al., Gene, 1998, 216(1): 55-65). As spacer sequences between the Lox sequences, m2 variants (Parrish et al., BioMed Research International, 2011, doi.org/10.1155/2011/924068) were used. In addition, the plasmid also had a Rox sequence arranged in the same direction as part of the CRE-loxP-like sequence. The Rox sequences are commonly known as target sequences for Dre recombinases, but have also been reported to cross Cre recombinases (Nat. Methods, 11 (2014), pp. 763-772). The marker sequence sandwiched by the Lox sequence in Plasmid 4 is inverted by the activity of CRE induced to express by the activation of the transcriptional regulatory sequence. The transcriptional regulatory sequence is a TCF/LEF DNA binding protein binding sequence and is activated by binding with TCF/LEF, and the activity of TCF/LEF is increased by activation of the Wnt/β-Catenin signaling pathway. Inversion of the marker sequence serves to reflect activation of the Wnt/β-Catenin signaling pathway in the cell into which the plasmid has been introduced.

### 5) Preparation of Viral Vector

Lentiviral vectors 1A, 1B, and 1C were prepared. The lentiviral vector 1A had the same shRNA (sh-scramble) as Plasmid 1 and a loxP-like sequence as a reporter system. The lentiviral vector 1B had the same shRNA (shRAD51) as Plasmid 2 and a loxP-like sequence as a reporter system. The loxP-like sequences of these vectors included two Lox2722-LoxP sequences arranged in opposite directions to each other and a red fluorescent protein mKate2 coding sequence arranged inverted therebetween. The lentiviral vector 1C had a p53RE transcriptional regulatory sequence and a CRE gene sequence. A vector set in which the lentiviral vector 1A or 1B and the lentiviral vector 1C were combined was constructed. When a DNA damage response in the cell is induced by the shRNA of the vector 1A or 1B, the vector set functions to sense the DNA damage response, and p53RE of the vector 1C is activated to express CRE, thereby causing a alteration in loxP-like sequence of the vector 1A or 1B (inversion of mKate2 sequence). The viral vectors 1A, 1B, and 1C were prepared by performing plasmid synthesis and lentivirus packaging at VectorBuilder Japan, Inc.

### 6) Preparation of Linear DNA 1

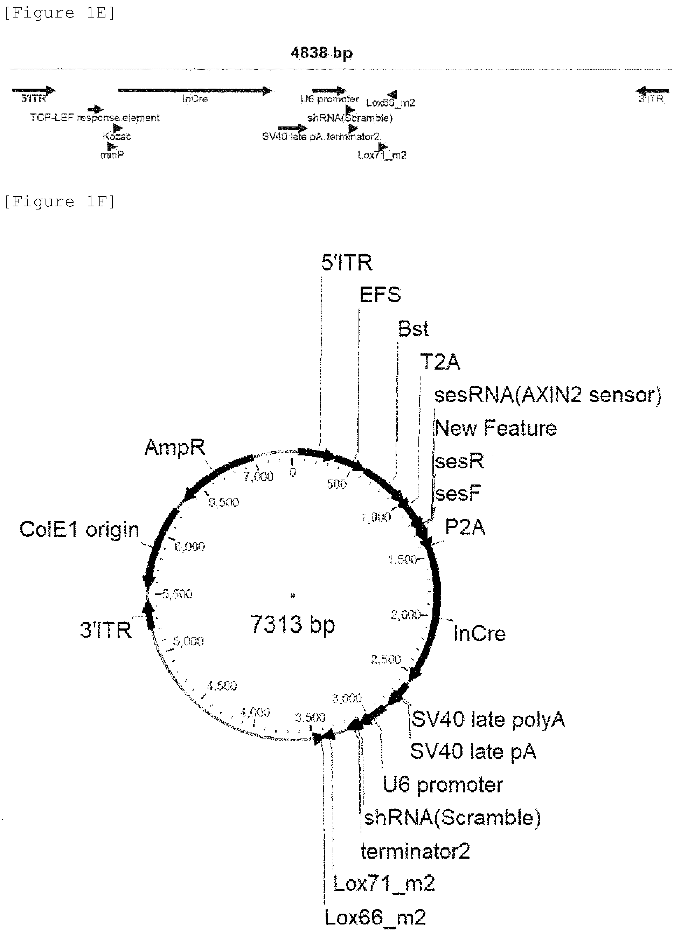

PCR with primers shown in Table A1 was performed using Plasmid 4 as a template, and the obtained fragment was purified using Wizard^{™} SV Gel and PCR Clean-Up System (Promega). The obtained DNA fragment was defined as the linear DNA 1. The linear DNA 1 had a region of a functional molecule including sh-scramble similar to Plasmid 4, transcriptional regulatory sequences, a reporter system, and sequences for causing these sequences to function in cells (Fig. 1E).

### 7) Preparation of Linear DNA 2

Linear DNAs including sequences encoding various shRNAs were prepared based on the linear DNA 1, and these were pooled to be used as the linear DNA 2. As the shRNA, an shRNA targeting a gene related to the Wnt pathway was selected. The specific sequence was prepared as follows. PCR was implemented using the primers shown in Table A2 with Plasmid 4 as a template, and the obtained PCR product was purified using the Wizard^{™} SV Gel and PCR Clean-Up System (Promega) to prepare a DNA fragment in which the region including the shRNA had been removed from the linear DNA 1. A pool of single-strand oligo DNA fragments containing sequences encoding shRNAs targeting genes related to the Wnt pathway (USP7, DHX29, or SETDB1) (Table B) was prepared by requesting Integrated DNA Technologies, Inc. The gene related to the Wnt pathway was a gene in which the Wnt pathway is activated by knockout (Evron et al., Oncogenesis, 2021, 10(9): 63). These DNA fragments were designed to overlap each other. These DNA fragments were mixed to form a template, and the fragments were bound by PCR. The obtained PCR product was electrophoresed with 1% of an agarose gel, and then a band of the fragment was cut out and purified by Wizard^{™} SV Gel and PCR Clean-Up System (Promega) to obtain a linear DNA 2A. The purified solution of the linear DNA 2A and the solution of the linear DNA 1 were mixed to obtain the linear DNA 2. The linear DNA 2 had a sequence encoding shRNA targeting the Wnt pathway or a functional molecular region including sh-scramble, a transcriptional regulatory sequence and a reporter system similar to Plasmid 4, and a sequence for causing these sequences to function in a cell, and was a DNA fragment population in which the functional molecular region was hetero.

### 8) Preparation of Linear DNA 3

DNA encoding sesRNA was incorporated into the transcription control region of the linear DNA 2 to prepare a linear DNA 3. The sesRNA is a module that expresses downstream reporters by any mRNA molecule and the activity of the ADAR protein in the cell (Nature, 2022, 610: 713-721, Nature Biotechnology, 2023, 41: 698-707, Nature Biotechnology, 2023, 41: 482-487, WO/2023/077135, WO/2023/077138, US20230123513). The DNA encoding sesRNA sensing mRNA of AXIN2 gene was designed. Since AXIN2 is increased in expression by activation of the Wnt pathway, the sesRNA increases in expression of downstream genes in response to activation of the Wnt pathway in the cell.

Plasmid 5 (Fig. 1F) was prepared by replacing the transcription control region of Plasmid 4 with the DNA encoding the sesRNA. Plasmid 5 was prepared by GenScript Japan Co., Ltd. Plasmid 5 was linearized by PCR using primers in Table C1 to obtain a linear DNA3 A. Next, the PCR was implemented using the primers shown in Table C2 with Plasmid 5 as a template, and the obtained PCR product was purified using the Wizard^{™} SV Gel and PCR Clean-Up System (Promega) to prepare a DNA fragment in which the region including the shRNA had been removed from the linear DNA 3A. A pool of single-stranded DNA fragments containing sequences encoding shRNAs (Table B) targeting genes related to the Wnt pathway was prepared by requesting Integrated DNA Technologies, Inc. These DNA fragments were designed to overlap each other. These DNA fragments were bound by NEBuilder (New England Biolabs Japan, Inc.). The PCR was performed using the bound fragment as a template, and the PCR product was purified by Wizard^{™} SV Gel and PCR Clean-Up System (Promega) to obtain a linear DNA 3B. The solution of the linear DNA 3A and the solution of the linear DNA 3B were mixed to obtain the linear DNA 3. The linear DNA 3 includes a functional molecular region including a sequence encoding an shRNA targeting the Wnt pathway or an sh-scramble, a sequence encoding sesRNA translational regulatory sequence and a reporter system similar to those of Plasmid 5, and a sequence for enabling these sequences to function in cells, and was a DNA fragment population in which the functional molecular region was hetero.

### Example 2 Response of Plasmid to DNA Damage Response

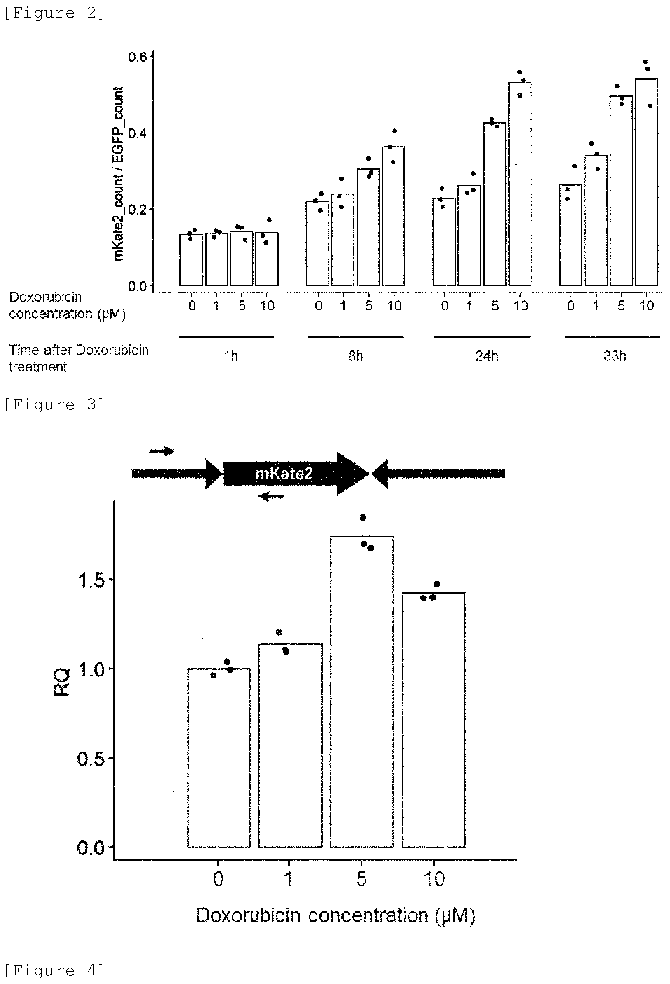

In order to verify the detectability of the DNA damage response by the plasmid prepared in Example 1, the reaction of Plasmid 1 to the artificially induced DNA damage response was examined. HEK293 cells were seeded at a concentration of 15,000 cells/well in a 96 well plate, and cultured in EMEM (with NEAA added) medium for 1 day. 100 ng/well of Plasmid 1 was introduced into cells using Lipofectamine 3000 kit (ThermoFisher). The amounts of both the P3000 reagent and the Lipofectamine 3000 reagent used for introduction were 0.2 µL/well. 40.5 hours after transfection, doxorubicin (Sigma-Aldrich), which has a DNA damaging effect, was added at a final concentration of 10 µM, 5 µM, 1 µM, or 0 µM (DMSO). Cells were observed with a 4x objective lens (BZ-PF04P, KEYENCE CORPORATION) using a fluorescence microscope BZ-X710 (KEYENCE CORPORATION) before and at 8, 24 and 33 hours after addition of doxorubicin. Green fluorescence by EGFP was detected by a filter set having an excitation wavelength of 470±20 nm and a fluorescence wavelength of 525±25 nm, and red fluorescence by mKate2 was detected by a filter set having an excitation wavelength of 545±12.5 nm and a fluorescence wavelength of 605±35 nm. Positive or negative determination of fluorescence was implemented using the hybrid cell count function implemented in the BZ-x Analyzer software from KEYENCE CORPORATION. As a result of the analysis, it was suggested that the value of the number of red fluorescence-positive cells/the number of green fluorescence-positive cells increased depending on the treatment time and concentration of doxorubicin (Fig. 2), and p53RE and CRE in the plasmid were activated by induction of DNA damage response by doxorubicin, so that inversion of the mKate2 sequence proceeded. From this result, it was shown that the plasmid of Example 1 can detect the DNA damage response of cells.

In order to confirm the inversion of the mKate2 sequence, cells were separated from the culture 33 hours after the doxorubicin treatment, and a DNA solution was obtained from the cells by phenol/chloroform extraction and ethanol precipitation. qPCR analysis by SYBR Green method was implemented using the primers shown in Table 4 and TB Green^{™} Premix Ex Taq^{™} II (Takara) to quantify the inverted sequence. As a result, the inversion rate (RQ) of the mKate2 sequence was increased by the doxorubicin treatment (Fig. 3), and a result corresponding to the above-described fluorescence assay was obtained.

### Example 3 Enhanced DNA Damage Response by Plasmid-encoded shRNA

The effect of enhancing the DNA damage response by the shRNA encoded by the plasmid prepared in Example 1 and the detectability of the effect by the plasmid were examined. As plasmids, Plasmid 1 having scrambled shRNA and Plasmid 2 having shRAD51 were used. Plasmids 1 and 2 both carry p53RE and express CRE recombinase due to DNA damage response. RAD51 suppressed by the shRNA of Plasmid 2 is a protein involved in DNA repair, and its expression suppression enhances the DNA damage response (Journal of medical chemistry, 2012, 55 (7): 3011-3020; Oncology Reports, 2019, 42 (6): 2426-2434).

HEK293 cells were seeded at a concentration of 25,000 cells/well in a 96 well plate, and cultured in EMEM (with NEAA added) medium for 1 day. Using Lipofectamine 3000 kit (ThermoFisher), 100 ng/well of Plasmid 1 and Plasmid 2 were each introduced into cells of different wells. The amounts of both the P3000 reagent and the Lipofectamine 3000 reagent used for introduction were 0.2 µL/well. 36 hours after transfection, doxorubicin (Sigma-Aldrich), which has a DNA damaging effect, was added at a final concentration of 5 µM or 0 µM (DMSO). Cells were observed in the same procedure as in Example 2 before addition of doxorubicin and at 26 hours and 74 hours after addition, and fluorescence positive or negative cells were counted. As a result of the analysis, after 74 hours from the addition of doxorubicin, the proportion of red fluorescence-positive cells in the wells including the shRAD51 (Plasmid 2)-introduced cells increased as compared with the wells containing the sh-scramble (Plasmid 1)-introduced cells (Fig. 4). This result showed that the plasmid-encoded shRAD51 enhanced the DNA damage response by doxorubicin, and the enhanced DNA damage response was detected by the plasmid.

### Example 4 Detection of Antioxidant Stress Response

Plasmid 3 with an ARE reactive to oxidative stress as a transcriptional regulatory sequence activated by a response in a cell, prepared in Example 1, was used to examine the response of the plasmid to artificially induced intracellular oxidative stress. HEK293 cells were seeded at a concentration of 25,000 cells/well in a 96 well plate, and cultured in EMEM (with NEAA added) medium for 1 day. 100 ng/well of Plasmid 3 was introduced into cells using Lipofectamine 3000 kit (ThermoFisher). The amounts of both the P3000 reagent and the Lipofectamine 3000 reagent used for introduction were 0.2 µL/well. 66 hours after transfection, tert-Butylhydroquinone (TBHQ, Tokyo Chemical Industry Co., Ltd.) was added at final concentrations of 100 µM, 50 µM, and 0 µM (DMSO) to induce oxidative stress. At 26 hours, 75 hours, and 99 hours after addition of TBHQ, cells were observed in the same procedure as in Example 2. Determination of fluorescent positive or negative cells was implemented using CellProfiler (Broad Institute [URL: cellprofiler.org/interfaces]). The analysis showed that the ratio of red fluorescent positive cells increased in a TBHQ treatment time- and concentration-dependent manner, suggesting that oxidative stress caused by TBHQ activated the ARE in the plasmid, which promoted inversion of the mKate2 sequence. It was shown that Plasmid 3 can detect oxidative stress in cells.

### Example 5 Detection of Wnt Signaling Pathway Activation

Plasmid 4 and the linear DNA 1, prepared in Example 1, having a TCF/LEF DNA binding protein binding sequence responsive to activation of the Wnt pathway as transcriptional regulatory sequences were used to examine the response of the vector to activation of the Wnt pathway in cells. HEK293 cells were seeded at a concentration of 15,000 cells/well in a 96 well plate, and cultured in EMEM (with NEAA added) medium for 1 day. Using Lipofectamine 3000 kit (ThermoFisher), 25 ng/well of Plasmid 4 and the linear DNA 1 were each introduced into cells. The amounts of both the P3000 reagent and the Lipofectamine 3000 reagent used for introduction were 0.2 µL/well. Three days after transfection, CHIR99021 (Cayman Chemical), that is a GSK3β inhibitor, was added at final concentrations of 16 µM, 8 µM, 4 µM, 2 µM, and 0 µM (DMSO) to activate the Wnt pathway.

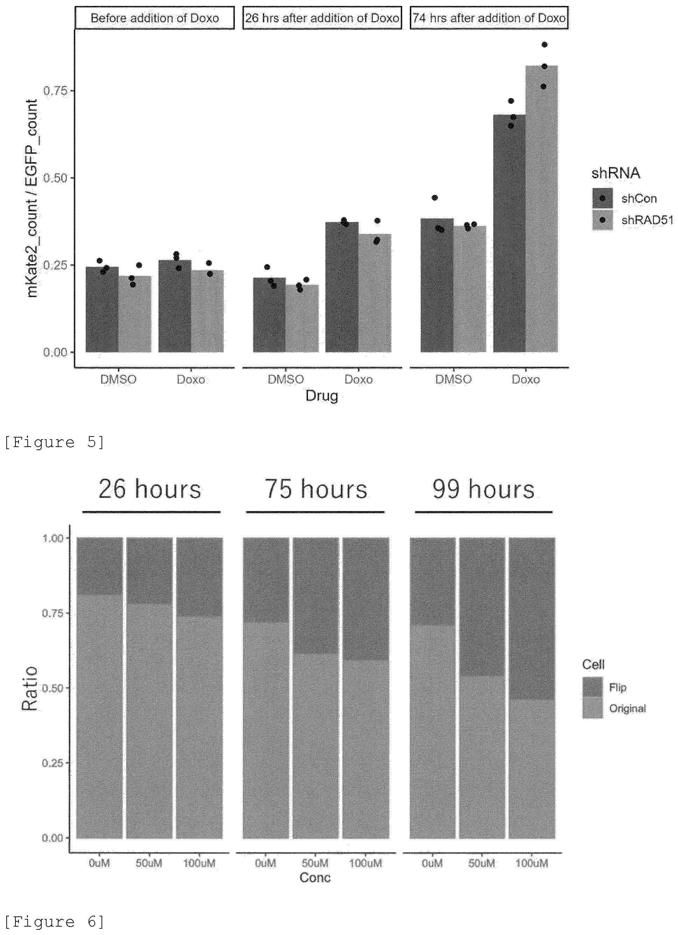

Activation of the Wnt pathway in cells after drug treatment was confirmed. After 6 hours and 24 hours from the drug treatment, RNA was extracted from the cells using SuperPrep^{™} Cell Lysis & RT Kit for qPCR (Toyobo Co., Ltd.), and cDNA was synthesized. The mRNA expression levels of AXIN2 and SP5 known as downstream genes of the Wnt pathway were quantified by RT-qPCR. This analysis was implemented by SYBR Green method using TB Green^{™} Premix Ex Taq^{™} II (Takara). GAPDH was used as an internal standard gene. The primers used for the analysis are shown in Table D. As a result of the analysis, under the condition that CHIR99021 was added at a final concentration of 16 µM and 8 µM at both 6 hours and 24 hours after the drug treatment, an increase in the mRNA expression levels of AXIN2 and SP5, which are known as downstream genes of the Wnt pathway, was observed, and it was confirmed that activation of the Wnt pathway occurred in cells by the drug addition (Fig. 6).

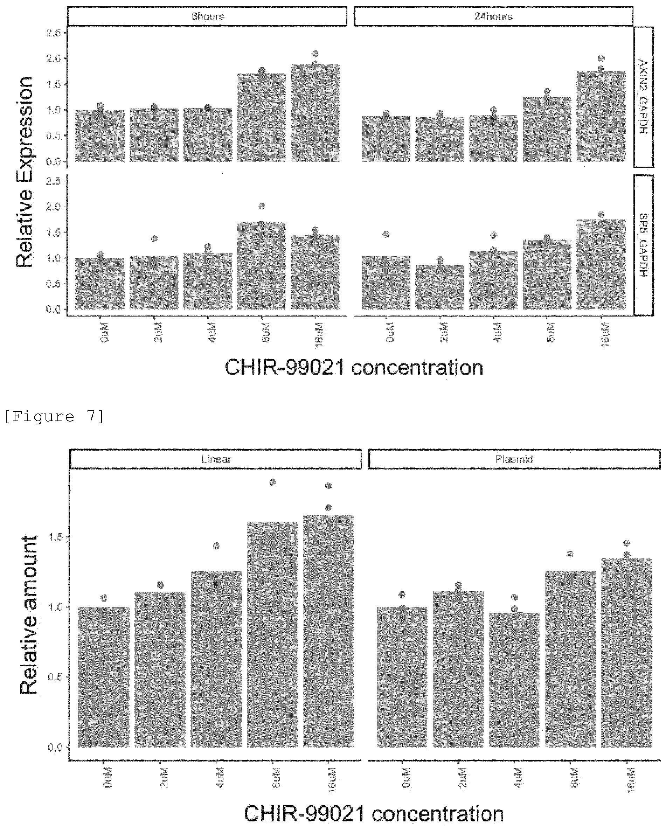

Next, the response of the vector to activation of the Wnt pathway was examined. 24 hours after the drug treatment, the cells were separated from the culture, and a DNA solution was obtained from the cells according to the manual using a Template Prepper for DNA (NIPPON GENE CO., LTD.) kit. Using the obtained DNA solution as a template, inversion of a marker sequence (m2 variant) was quantified by qPCR analysis by SYBR Green method using TB Green^{™} Premix Ex Taq^{™} II (Takara). The primers used for the analysis are shown in Table E. Quantitative values of backbone sequences were used for normalization. As a result of the analysis, an increase in the CHIR99021 concentration-dependent inverted sequence was detected in both Plasmid 4 introduction group and the linear DNA 1 introduction group (Fig. 7). It was shown that the vector of the present invention can detect the Wnt pathway activation in the cell. In addition, the results of Examples 2 to 5 showed that various intracellular responses can be detected by changing the type of transcriptional regulatory sequence in the plasmid.

### Example 6 Detection of Intracellular Response by Viral Vector and shRNA Functional Analysis

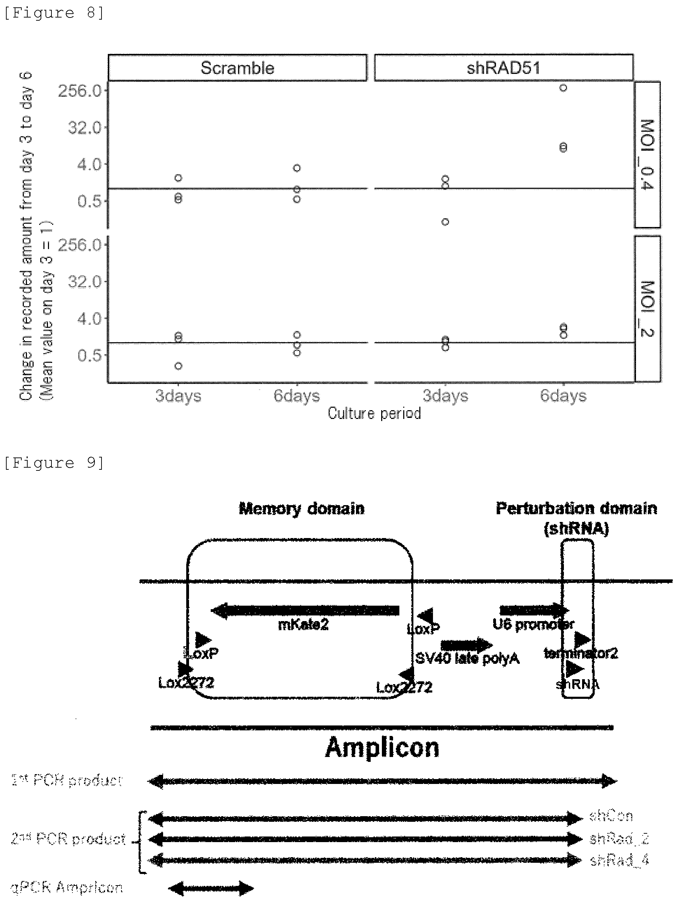

The viral vector set prepared in Example 1 was examined for the ability to induce an intracellular response by shRNA and the ability to detect the intracellular response. HEK293 cells were seeded at a concentration of 15,000 cells/well in a 96 well plate, and cultured in EMEM (with NEAA added) medium for 1 day. For each culture well, the viral vector 1A or 1B was introduced so that the multiplicity of infection (MOI) was 0.4 or 2, and the viral vector 1C was introduced at the MOI of 10. 72 and 144 hours after transduction, the cells were separated from the culture, and a DNA solution was obtained from the cells according to the manual using a Template Prepper for DNA (NIPPON GENE CO., LTD.) kit. Using the obtained DNA solution as a template, a region including mKate2 between Lox sequences in the vector was amplified by PCR using primers in Table F. Inversion of the mKate2 region within the PCR product was quantified by qPCR analysis by SYBR Green method using TB Green^{™} Premix Ex Taq^{™} II (Takara). For quantification, primers described in Table 4 were used, and the amount ratio between the inverted mKate2 region and the non-inverted mKate2 region was calculated. As a result, the inversion of the mKate2 sequence was increased in the vector including shRAD51 between 72 and 144 hours after transduction (Fig. 8). This result showed that shRAD51 mounted on the viral vector inhibited the expression of RAD51 to induce a DNA damage response in a cell, and this intracellular response was detected by the reporter system of the vector set. In addition, from the results of Examples 2 to 6, it was shown that detection of intracellular responses generated by shRNAs can be performed with various vectors.

### Example 7 Comprehensive Detection of Enhancement Effect of DNA Damage Response by Various shRNAs included in Plasmid Pool

It was verified that even when different plasmids were pooled and introduced into cells, each reporter signal depending on the shRNA encoded by each plasmid could be detected. As plasmids, Plasmid 1 including scrambled shRNA and Plasmid 2 including a pool of four types of plasmids having different shRAD51 sequences were used.

A plasmid mixture including Plasmid 1 and Plasmid 2 in an equimolar ratio was prepared. HEK293 cells were seeded at a concentration of 25,000 cells/well in a 96 well plate, and cultured in EMEM (with NEAA added) medium for 1 day. Using Lipofectamine 3000 kit (ThermoFisher), the plasmid mixture including Plasmids 1 and 2 at a total of 25 ng/well was introduced into cells. The amounts of both the P3000 reagent and the Lipofectamine 3000 reagent used for introduction were 0.2 µL/well. 72 hours after transfection, doxorubicin (Sigma-Aldrich), which has a DNA damaging effect, was added at a final concentration of 1 µM and 0 µM (DMSO).

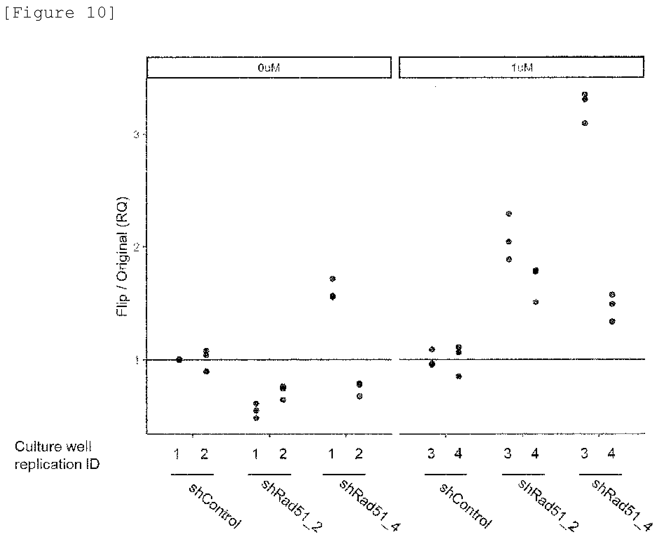

96.5 hours after the doxorubicin treatment, the cells were separated from the culture, and a DNA solution was obtained from the cells according to the manual using a Template Prepper for DNA (NIPPON GENE CO., LTD.) kit. Then, from the DNA solution, a fragment including the shRNA region and the mKate2 coding sequence was amplified by PCR using the primers in Table 5. Next, amplicons including the mKate2 sequence were prepared for each shRNA region by PCR using the primers in Table 6 (Fig. 9). The inversion amount of the mKate2 sequence included in the amplicon including sh-scramble, shRAD51_2, and shRAD51_4 was quantified by qPCR analysis by SYBR Green method using primers in Table 7 and TB Green^{™} Premix Ex Taq^{™} II (Takara). As a result of the analysis, in the cells to which doxorubicin was added, in the amplicon including shRAD51_2 and shRAD51_4, the inversion rate of the mKate2 sequence was increased as compared with the amplicon including sh-scramble (Fig. 10) .

In order to confirm the results by an analysis method different from qPCR, the fragment including the shRNA region and the mKate2 sequence was analyzed using massively parallel sequencing. Adaptors were added to the amplicons using a Ligation Sequencing Kit (Nanopore, SQK-LSK109) to create a library for Nanopore analysis, and then sequencing was implemented using a Flongle Flow Cell (Nanopore). Next, reference sequence data covering combinations of mKate2 sequences and shRNA sequences that can occur theoretically was created, and reads were mapped by BWA-MEM (arXiv: 1303.3997[q-bio.GN], 2013). From the obtained results, the inverted and non-inverted mKate2 sequences were counted for each shRNA, and the odds of the inversion of the mKate2 sequence in each shRNA were calculated. Next, the relative odds of each shRNA when the odds of the sh-scramble were set to 1 were calculated. As a result of the analysis, the mKate2 sequence on the same read as shRAD51 had an increased inversion rate (Table 8). From these results, it was shown that even when a plurality of plasmids including different shRNAs are pooled and introduced into cells, the effect of the response in the cell caused by each shRNA can be detected at once.

**[Table 8]**

| Table 8 Analysis Result of mKate2 Sequences Present in the same Read as e ach shRNA | | | | |
|---|---|---|---|---|
| **shRNA** | **Original Memory Sequence (Count)** | **Flipped Memory Sequence (Count)** | **Odds** | **Odds Ratio** |
| **shScramble** | 8389 | 37030 | 4.414 | 1.000 |
| **shRAD51_1** | 11370 | 51238 | 4.506 | 1.021 |
| **shRAD51_2** | 11835 | 54862 | 4.636 | 1.050 |
| **shRAD51_3** | 14252 | 66833 | 4.689 | 1.062 |
| **shRAD51_4** | 10122 | 47320 | 4.675 | 1.059 |

### Example 8 Searching for shRNAs that produce Intracellular Response using Vector Pool

It was verified that the vector of the present invention can search for and identify shRNAs that activate the Wnt pathway. As a vector, the linear DNA 2 prepared in Example 1 was used. The linear DNA 2 was a pooled DNA fragment including the linear DNA with shRNA and sh-scramble linear DNA that inhibit genes related to the Wnt pathway (SETDB1, DHX29, or USP7, whose inhibition activates the Wnt pathway). HEK293 cells were seeded at a concentration of 15,000 cells/well in a 96 well plate, and cultured in EMEM (with NEAA added) medium for 1 day. Using Lipofectamine 3000 kit (ThermoFisher), 25 ng/well of the linear DNA 2 in total was introduced into cells. The amounts of both the P3000 reagent and the Lipofectamine 3000 reagent used for introduction were 0.2 µL/well.

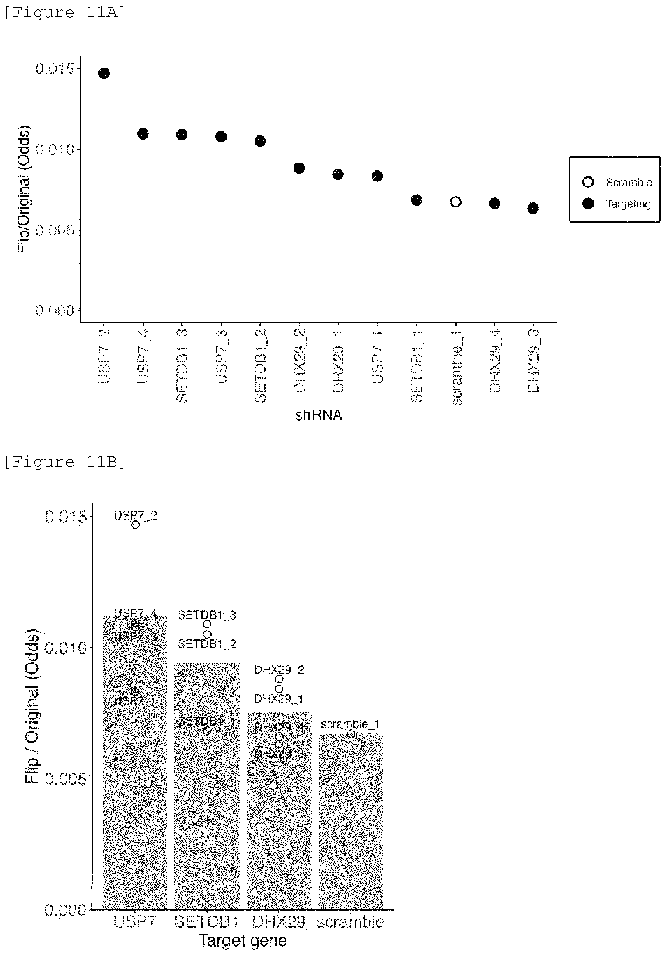

72 hours after the lipofection, the cells were separated from the culture, and a DNA solution was obtained from the cells according to the manual using a Template Prepper for DNA (NIPPON GENE CO.**,** LTD.) kit. Using the DNA solution as a template, PCR using primers in Table G was performed to amplify a region including a marker sequence (sequence between Lox sites) and shRNA coding sequence. Since an index sequence was added to a terminal of the primer used, an index corresponding to each culture well was given to the obtained amplicon. Adapters were added to the obtained amplicons using a Ligation Sequencing Kit (Nanopore, SQK-LSK110) to prepare a library for Nanopore analysis, and sequencing was implemented using a Flongle Flow Cell (Nanopore). The obtained reads were subjected to index identification and removal by Minibar (github.com/calacademy-research/minibar). Next, a series of reference sequence data covering combinations of marker sequences and shRNA sequences that can occur theoretically was created, and reads were mapped to the reference sequences using BWA-MEM. From the obtained results, the presence or absence of inversion of the marker sequence for each shRNA was counted, and the odds of inversion for each shRNA were calculated. As a result of the analysis, marker sequences arranged on the same read as shRNA targeting genes related to the Wnt pathway had higher odds of inversion compared to those arranged on the same read as scrambled shRNA (Fig. 11). From these results, it has been demonstrated that the vector of the present invention can identify shRNAs that activate the Wnt pathway, and that even if a plurality of vectors including different shRNAs are pooled and introduced into cells, the effects of the responses in the cell caused by each shRNA can be detected simultaneously.

In addition, the score of the marker sequence present in the same molecule as the shRNA was counted for each target gene to examine the effect of gene perturbation on the response in the cell. The data in Fig. 11A was tabulated for each target gene, and the average value was calculated (Fig. 11B). As a result, the mean value of the inversion odds of the vector inhibiting each target gene (particularly USP7 and SETDB1) increased compared to the control. It was suggested that these target genes contribute to suppression of activation of the Wnt/β-Catenin signaling pathway in a cell. From the above, by collectively analyzing the results for each gene as a target of functional molecules, genes associated with responses in the cells could be evaluated and ranked.

### Example 9 Searching for shRNAs that produce Intracellular Response using Vector Pool with Different Detection Systems

It was verified that the linear DNA 3 prepared in Example 1 can search for and identify shRNA that activates the Wnt pathway. Similar to the linear DNA 2, the linear DNA 3 was a pooled DNA fragment including the linear DNA with the shRNA that inhibits genes related to the Wnt pathway and the linear DNA with sh-scramble. The linear DNA 3 had sequence encoding sesRNA as a translational regulatory region, and the sesRNA expressed from the linear DNA 3 increased the expression of downstream gene (CRE) according to the mRNA expression of AXIN2 downstream of the Wnt pathway, thereby detecting the activation of the Wnt pathway. HEK293 cells were seeded at a concentration of 15,000 cells/well in a 96 well plate, and cultured in EMEM (with NEAA added) medium for 1 day. A total of 5 ng/well of the linear DNA 3 was introduced to the cells using the Lipofectamine 3000 kit (ThermoFisher). The amounts of both the P3000 reagent and the Lipofectamine 3000 reagent used for introduction were 0.2 µL/well.

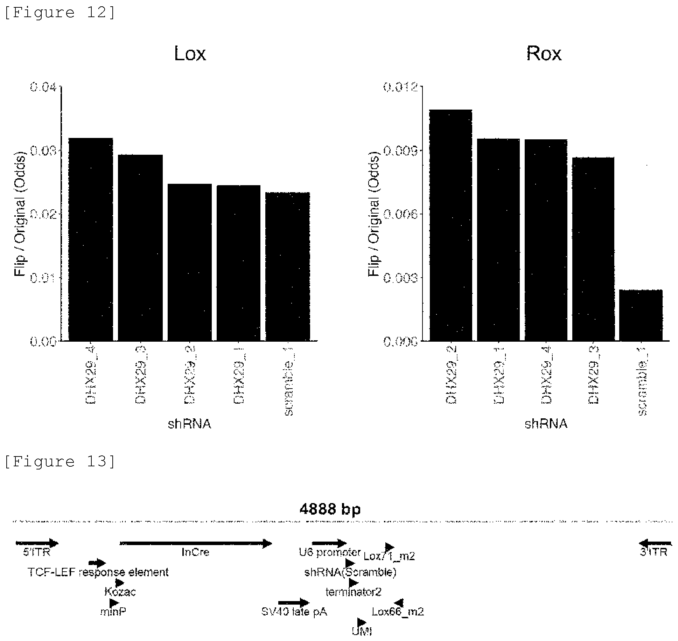

72 hours after the lipofection, the cells were separated from the culture, and a DNA solution was obtained from the cells according to the manual using a Template Prepper for DNA (NIPPON GENE CO., LTD.) kit. Using the DNA solution as a template, PCR using primers in Table G was performed to amplify a region including a marker sequence (sequence between Lox sites) and an shRNA sequence. Since an index sequence was added to a terminal of the primer used, an index corresponding to the experimental condition was given to the obtained amplicon. Adapters were added to the obtained amplicons using a Ligation Sequencing Kit (Nanopore, SQK-LSK110) to prepare a library for Nanopore analysis, and sequencing was implemented using a Flongle Flow Cell (Nanopore). The obtained reads were subjected to index identification and removal by Minibar. Next, a series of reference sequence data covering combinations of marker sequences and shRNA sequences that can occur theoretically was created, and reads were mapped to the reference sequences using BWA-MEM. From the obtained results, the presence or absence of inversion of the marker sequence was counted for each shRNA, and the inversion odds of the region flanked by Lox sequences and the deletion odds of the sequence flanked by Rox sequences in each shRNA were calculated. As a result of the analysis, marker sequences on the same read as shRNAs (DHX29_1 to DHX29_4) targeting genes related to the Wnt pathway had higher inversion odds and deletion odds than those on the same read as scrambled shRNA (Fig. 12). From these results, it was shown that the shRNA sequence capable of activating the Wnt pathway in the cell or the gene related to the Wnt pathway can be screened using the vector pool used in this example. In addition, from Examples 7 and 8, it was shown that the vector pool of the present invention can use a variety of transcriptional regulatory sequences and reporter systems in order to detect the effect of responses in the cells caused by shRNAs.

### Example 10 Improvement of Detection Capability by Introduction of UMI Sequence

### 1) Preparation of Linear DNA 4

The reporter system included in the vector was identified by an identifier (Unique Molecular Identifier; UMI) sequence. The linear DNA 4 (Fig. 13) in which a 50 bp UMI sequence was introduced between the shRNA coding sequence of the linear DNA 1 or 2 and the reporter system sequence was prepared. As a UMI sequence, a pool of random single-strand oligo DNAs was prepared by requesting FASMAC Co., Ltd.

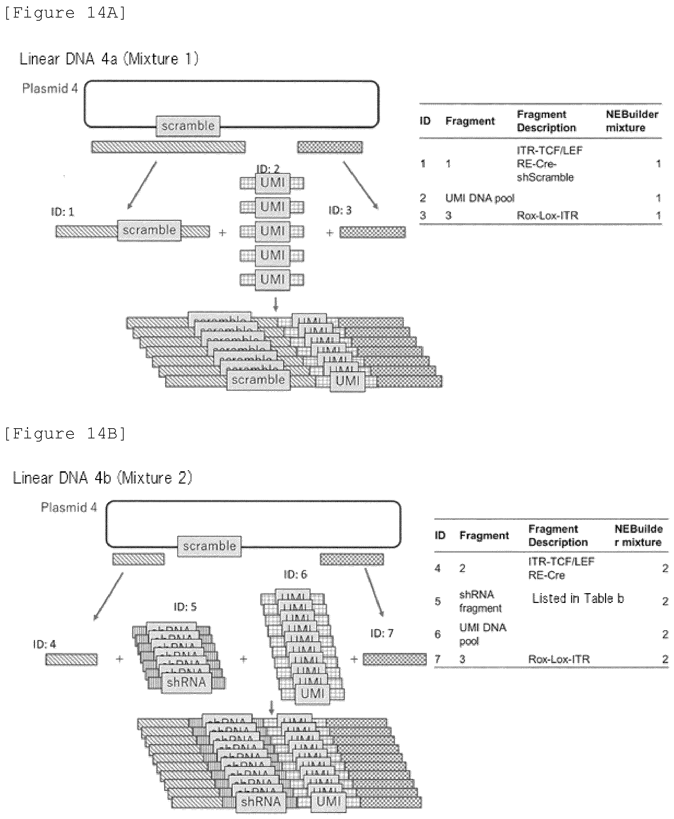

A pool of single-stranded DNA fragments containing sequences encoding shRNAs (Table B) targeting genes related to the Wnt pathway was prepared by requesting Integrated DNA Technologies, Inc. PCR was performed using the primers shown in Table H1 and Plasmid 4 as a template, and the resulting PCR product was purified using Wizard^{™} SV Gel and PCR Clean-Up System (Promega) to prepare a DNA fragment. These DNA fragments were designed to overlap each other. These DNA fragments and DNA fragments containing UMIs were mixed in the combination shown in Figs. 14A and 14B, and bound by NEBuilder (New England Biolabs Japan Ltd.) to obtain a linear DNA 4A including sh-scramble and a linear DNA 4B including shRNA targeting a Wnt-related gene. PCR was performed using the linear DNAs 4A and 4B as templates and the primers in Table H2, and the PCR products were purified using the Wizard^{™} SV Gel and PCR Clean-Up System (Promega). A solution of the linear DNA 4A and a solution of the linear DNA 4B were mixed to obtain the linear DNA 4. The linear DNA 4 had a sequence encoding shRNA targeting the Wnt pathway or a functional molecular region including sh-scramble, a transcriptional regulatory sequence and a reporter system similar to Plasmid 4, and a sequence for causing these sequences to function in a cell, and was a DNA fragment population in which the functional molecular region was heterogeneous. Each DNA fragment in the linear DNA 4 included a UMI sequence for identifying an individual fragment.

### 2) Preparation of Plasmid 6

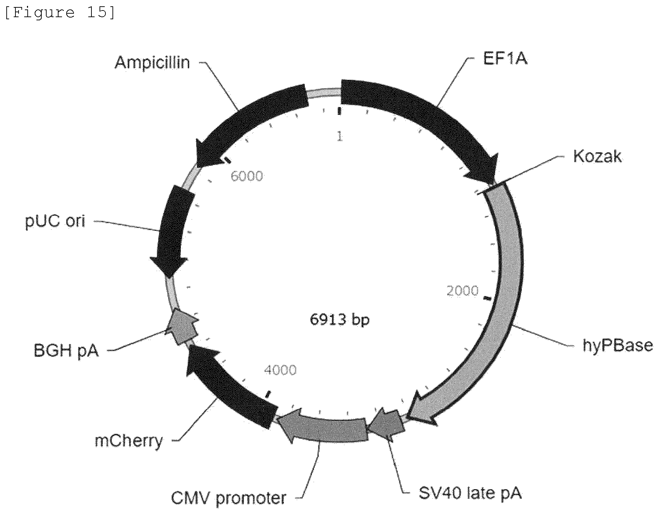

A hyperactive piggyBac transposase (hyPBase) expression plasmid was designed as shown in Fig. 15 and produced by VectorBuilder Japan, Inc. The obtained plasmid was designated as Plasmid 6.

### 3) Verification of Effect of UMI Sequence

HEK293 cells were seeded at a concentration of 15,000 cells/well in a 96 well plate, and cultured in EMEM (with NEAA added) medium for 1 day. Using Lipofectamine 3000 kit (ThermoFisher), 5 ng/well of the linear DNA 4 in total and 95 ng/well of Plasmid 6 were simultaneously introduced into cells. The linear DNA 4 has an ITR sequence (Fig. 13), which is incorporated into the genome by the activity of the hyPBase expressed from Plasmid 6. The linear DNA 4 is a DNA fragment obtained by pooling linear DNAs each having shRNA that inhibits a gene related to Wnt and a scrambled shRNA. The amounts of both the P3000 reagent and the Lipofectamine 3000 reagent used for introduction were 0.2 µL/well.

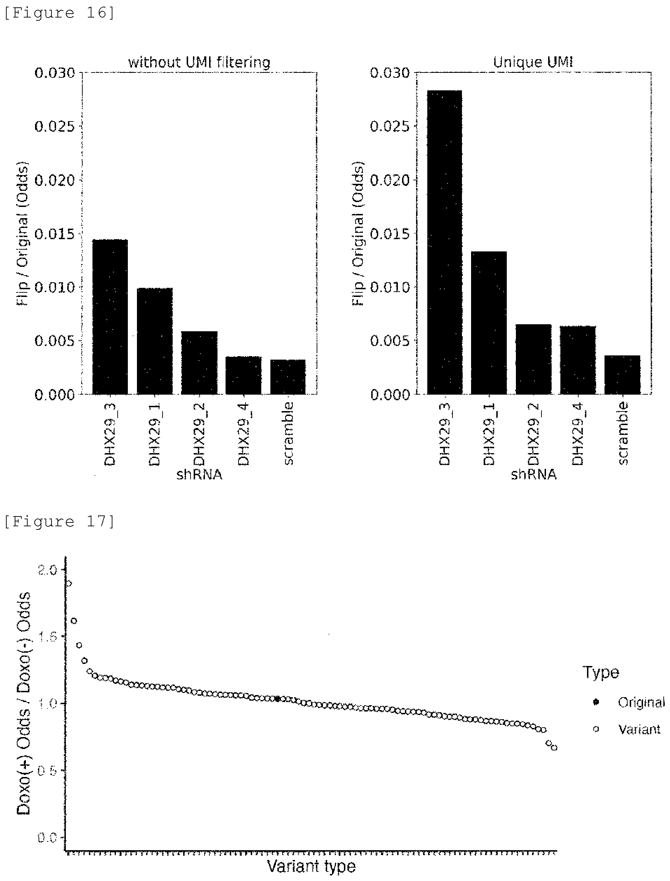

72 hours after the lipofection, the cells were separated from the culture, and a DNA solution was obtained from the cells according to the manual using a Template Prepper for DNA (NIPPON GENE CO., LTD.) kit. Using the DNA solution as a template and the primers in Table G, a region including the marker sequence (Lox intersequence), UMI, and shRNA sequence was amplified. Since an index sequence was added to a terminal of the primer used, an index corresponding to the experimental condition was given to the obtained amplicon. Adapters were added to the obtained amplicons using a Ligation Sequencing Kit (Nanopore, SQK-LSK110) to prepare a library for Nanopore analysis, and sequencing was implemented using a Flongle Flow Cell (Nanopore). The obtained reads were subjected to index identification and removal by Minibar. Next, a series of reference sequence data covering combinations of marker sequences and shRNA sequences that can occur theoretically was created, and reads were mapped to the reference sequences using BWA-MEM. In addition, UMI regions were identified from the SAM file and read information obtained by mapping, and UMI sequences were clustered by CD-HIT to determine whether the obtained UMI was unique. The presence or absence of inversion of the marker sequence was counted for each read, and the case in which filtering by UMI sequence was not performed and the case in which analysis was performed only with reads with unique UMI sequences, and the inversion odds is calculated in each shRNA (sh-scramble, DHX29_1 to DHX29_4). As a result of the analysis, marker sequences on the same read as shRNAs targeting Wnt-related genes (DHX29_1 to DHX29_4) had higher inversion odds than those on the same read as scrambled shRNA, and this tendency was even more pronounced when only reads with unique UMIs were analyzed (Fig. 16). From these results, it was considered that by introducing the UMI into the reporter system, bias associated with sequence amplification at the time of analysis can be avoided, and the detection capability of the target shRNA by the vector of the present invention is enhanced.

Example 11 Activity Evaluation of Transcriptional Regulatory Sequence

### 1) Preparation of Plasmid 7

A plasmid vector (Plasmid 7) was prepared in the same procedure as in Plasmid 1 except that PCR was implemented using a combination of a template and a primer shown in Table I, and the molecular weight of the plasmid vector was confirmed. The minP variant sequences provided as templates were a pool of 94 minP variant sequences, including the original sequence. This plasmid had sh-scramble in the functional molecular region, p53RE as a transcriptional regulatory sequence, and a CRE-loxP-like sequence as a reporter system. The CRE-loxP-like sequence had a CRE gene sequence and two Lox2722-LoxP sequences arranged in opposite directions to each other with the mKate2 sequence arranged therebetween. The mKate2 sequence was inverted, transcribed, and expressed by the action of CRE when the transcriptional regulatory sequence was activated (see Fig. 1B). Plasmid 7 had a diverse minimal promoter (minP variant) sequence located between p53RE and CRE, providing a pool of vectors each having a total of 94 minP variant sequences.

### 2) Activity Evaluation of Transcriptional Regulatory Sequence

An evaluation system for evaluating the activity of a transcriptional regulatory sequence for an intracellular response was examined using Plasmid 7. HEK293 cells were seeded at a concentration of 25,000 cells/well in a 96 well plate, and cultured in EMEM (with NEAA added) medium for 1 day. A total of 25 ng/well of Plasmid 7 was introduced into the cells using Lipofectamine 3000 kit (ThermoFisher). The amounts of both the P3000 reagent and the Lipofectamine 3000 reagent used for introduction were 0.2 µL/well. 4 days after transfection, doxorubicin (Sigma-Aldrich), which has a DNA damaging effect, was added at a final concentration of 5 µM and 0 µM (DMSO).

24 hours after the doxorubicin treatment, the cells were separated from the culture, and a DNA solution was obtained from the cells according to the manual using a Template Prepper for DNA (NIPPON GENE CO.**,** LTD.) kit. Using the DNA solution as a template, a fragment including the minP variant sequence and the mKate2 sequence was amplified by PCR using the primers in Table J**.** Adapters were added to the obtained amplicons using a Ligation Sequencing Kit (Nanopore, SQK-LSK110) to prepare a library for Nanopore analysis, and then sequencing was performed using a Flongle Flow Cell (Nanopore). A series of reference sequence data items covering combinations of the mKate2 sequences and the minP variant sequences and sequences that can occur theoretically was created, and reads were mapped to the reference sequences using BWA-MEM. From the obtained results, inverted and non-inverted mKate2 sequences were counted for each minP variant sequence, and the odds of inversion of mKate2 sequences in each minP variant sequence were calculated. As a result of the analysis, the minP variant sequence that more sensitively reacts to stimulation by doxorubicin than the original minP sequence was detected (Fig. 17). From these results, it was shown that a vector system for evaluating the activity of vector components such as transcriptional regulatory sequences for intracellular responses can be constructed.

## Claims

1. A vector or set of vectors for analyzing a function of a functional molecule, comprising:
a polynucleotide encoding an expression system of a candidate molecule of a functional molecule;
a polynucleotide encoding a transcriptional regulatory sequence or translational regulatory sequence that is activated by a predetermined response in a cell; and
a polynucleotide encoding a reporter system operably linked to the transcriptional regulatory sequence or translational regulatory sequence.

2. The vector or set of vectors according to claim 1, wherein the functional molecule is RNA or a peptide.

3. The vector or set of vectors according to claim 2, wherein the RNA is shRNA or guide RNA**.**

4. The vector or set of vectors according to claim 1, wherein the transcriptional regulatory sequence or translational regulatory sequence that is activated by the predetermined response in the cell is a p53 binding sequence, an antioxidant responsive element, a nuclear factor kappa B binding sequence, an activating transcription factor 6 responsive sequence, a metal regulatory element, a heat shock element, a hypoxia responsive element, a xenobiotic responsive element, a nuclear factor of activated T-CELLS binding sequence, a TCF/LEF DNA binding protein binding sequence, sesRNA, and toehold RNA.

5. The vector or set of vectors according to claim 4, wherein the transcriptional regulatory sequence activates a promoter arranged downstream thereof.

6. The vector or set of vectors according to claim 5, wherein the reporter system includes the promoter.

7. The vector or set of vectors according to claim 1, wherein the reporter system is expressed in response to activation of the transcriptional regulatory sequence or translational regulatory sequence.

8. The vector or set of vectors according to claim 1, wherein the reporter system is a luminescent or fluorescent protein reporter system, or an enzymatic reporter system.

9. The vector or set of vectors according to claim 8, wherein the enzymatic reporter system includes a combination of a protein having an effect of altering a target nucleotide sequence and its target sequence.

10. The vector or set of vectors according to claim 8, wherein the enzymatic reporter system expresses a DNA nuclease, a DNA recombinase, a transposase, or an integrase.

11. The vector or set of vectors according to claim 8, wherein the enzymatic reporter system includes a combination of an artificial nuclease and its recognition sequence, and a combination of a restriction enzyme and a corresponding restriction enzyme recognition sequence.

12. The vector or set of vectors according to claim 8, wherein the enzymatic reporter system includes a combination of a Cas family, a guide RNA, and a Cas recognition sequence, a combination of a TALEN and a recognition sequence thereof, a combination of a ZFN and a recognition sequence thereof, or a combination of a base editor or prime editor and a recognition sequence thereof.

13. The vector or set of vectors according to claim 11, wherein the restriction enzyme is I-CeuI, I-SceI, PI-PspI, or PI-SceI.

14. The vector or set of vectors according to claim 8, wherein the enzymatic reporter system includes a CRE-LoxP system, a system having a sequence of a LoxP variant instead of or in addition to the LoxP sequence in the CRE-LoxP system, a Vika-Vox system, a Dre-rox system, or a Flp-FRT system.

15. The vector or set of vectors according to claim 1, wherein the vector is a plasmid vector, a linear DNA sequence, a transposon vector, or a viral vector.

16. The vector or set of vectors according to claim 15, wherein the viral vector is a lentiviral vector, an adeno-associated viral vector, a baculoviral vector, an MMLV retroviral vector, or an MSCV retroviral vector.

17. The vector or set of vectors according to claim 1, further comprising a unique molecular identifier sequence.

18. A method for searching a functional molecule causing a response in a cell, the method comprising:
introducing the vector or set of vectors according to any one of claims 1 to 17 into a cell; and
measuring expression of a reporter system included in the vector or set of vectors in the cell.

19. The method according to claim 18, wherein the expression of the reporter system is quantified by luminescence or fluorescence measurement, protein quantification, quantitative RT-PCR, quantitative PCR, or sequencing.

20. The method according to claim 18, further comprising:
adding two or more vectors or sets of vectors to a cell population to introduce each vector into any of the cells in the cell population, wherein each of the two or more vectors or sets of vectors includes a polynucleotide encoding a different functional molecule.

21. The method according to claim 18, wherein the cell is a cell collected from a human or a non-human animal, a cell derived from a specimen collected from a patient or a laboratory animal, a primary cultured cell, an organoid, a spheroid, an immortalized cultured cell, or a cell in a body of an individual of a laboratory animal.

22. A method for searching a gene involved in a response of a cell, the method comprising:
introducing a plurality of vectors or sets of vectors into a cell population, wherein each of the plurality of vectors or sets of vectors encodes
RNA that interferes with a gene of interest,
a transcriptional regulatory sequence or translational regulatory sequence that is activated by a predetermined response in a cell, and
a reporter system operably linked to the transcriptional regulatory sequence or translational regulatory sequence,
the RNA encoded by each of the plurality of vectors or sets of vectors being different from one another;
measuring expression of a reporter system encoded by each of the plurality of vectors or sets of vectors in the cell populations; and
determining whether the gene of interest is a gene involved in the predetermined response in the cell based on the expression of the reporter system included in each of the plurality of vectors or sets of vectors.

23. The method according to claim 22, wherein whether the gene of interest is a gene involved in the predetermined response in the cell is determined based on statistical value of the expression for the reporter system included in each of the plurality of vectors or sets of vectors.

24. The method according to claim 22, wherein the RNA that interferes with the gene of interest is shRNA or gRNA.

25. A reagent kit comprising the vector or set of vectors according to any one of claims 1 to 17.

26. The reagent kit according to claim 25, wherein the kit is a diagnostic agent for a disease or a companion diagnostic agent.

27. A method for diagnosing a disease, the method comprising implementing the method for searching a functional molecule causing a response in a cell according to any one of claims 18 to 21.

28. A companion diagnostic method comprising implementing the method for searching a functional molecule causing a response in a cell according to any one of claims 18 to 21.

29. A method for analyzing an activity of a transcriptional regulatory sequence or translational regulatory sequence for a response in a cell, the method comprising:
preparing a cell into which a vector has been introduced, wherein
the vector includes a polynucleotide encoding a transcriptional regulatory sequence or translational regulatory sequence, a polynucleotide encoding a reporter system operably linked to the transcriptional regulatory sequence or translational regulatory sequence, and a label sequence identifying the transcriptional regulatory sequence or translational regulatory sequence,
the reporter system includes a protein having an effect of changing a target nucleotide sequence and a target sequence thereof, and expression of the reporter system alters the recognition sequence,
in the cell, eliciting a predetermined response in the cell;
after eliciting the response, analyzing a region including the target sequence and the label sequence in the vector and quantifying the alteration in the target sequence;
associating the target sequence with the transcriptional regulatory sequence or translational regulatory sequence based on the label sequence; and
evaluating the activity of the transcriptional regulatory sequence or translational regulatory sequence associated with the target sequence against the predetermined response in the cell based on the amount of alteration in the target sequence.

30. The method according to claim 29, wherein the vector includes a polynucleotide encoding the transcriptional regulatory sequence and the label sequence identifying the transcriptional regulatory sequence.

31. The method according to claim 29, wherein the region including the target sequence and the label sequence is analyzed by sequencing.

32. The method according to claim 29, the method further comprising:
introducing a plurality of vectors into a cell population, wherein
the plurality of vectors each include a polynucleotide encoding a different transcriptional regulatory sequence or translational regulatory sequence.
